# FASCICULE DE BREVET EUROPEEN

(11) **EP 2 439 193 B1**
(45) Date de publication et mention de la délivrance du brevet: **23.04.2014**
(21) Numéro de dépôt: 11188580.2
(22) Date de dépôt: 09.08.2007
(51) Int. Cl.: C07D 213/60, C07D 241/10, C07D 401/04, A61K 31/435, A61P 9/00

(54) **Dérivés de 5,6-bisaryl-2-pyridine-carboxamide, leur préparation et leur application en thérapeutique comme antagonistes des recepteurs a l'urotensine II**
5,6-Bisaryl-2-Pyridin-Carboxamid-Derivate, ihre Herstellung und therapeutische Anwendung als Urotensin-II-Rezeptorantagonisten
Derivatives of 5,6-bisaryl-2-pyridine-carboxamide, preparation and use thereof in therapeutics as antagonists of urotensin II receptors

(30) Priorité: 11.08.2006 FR 0607283
(43) Date de publication de la demande: 11.04.2012
(62) Demande divisionnaire de: 07823409.3
(73) Titulaire: SANOFI, 75008 Paris (FR)
(72) Inventeur: Altenburger, Jean-Michel, 75013 Paris (FR); Fossey, Valerie, 75013 Paris (FR); Lassalle, Gilbert, 75013 Paris (FR); Petit, Frédéric, 75013 Paris (FR); Vernieres, Jean-Claude, 75013 Paris (FR); Janiak, Philip, 75013 Paris (FR)
(74) Mandataire: Essler, Frank

(56) Documents cités:
- US-A- 4 916 145
- JEFFERSON W T ET AL: "Biphenylcarboxamide Derivatives as Antagonists of Platelet-Activating Factor", JOURNAL OF MEDICINAL CHEMISTRY, AMERICAN CHEMICAL SOCIETY, US, vol. 32, no. 8, 1 janvier 1989 (1989-01-01), pages 1814-1820, XP002222168, ISSN: 0022-2623, DOI: 10.1021/JM00128A025
- BRENNA E ET AL: "New route to o-terphenyls: application to the synthesis of 6,7,10,11-tetramethoxy-2-(methoxycarbonyl) triphenylene", JOURNAL OF THE CHEMICAL SOCIETY, PERKIN TRANSACTIONS 1, CHEMICAL SOCIETY, LETCHWORTH; GB, no. 5, 1 janvier 1998 (1998-01-01), pages 901-904, XP002221111, ISSN: 0300-922X, DOI: 10.1039/A707738F

## Description

La présente invention a pour objet des intermédiaires des dérivés de 5,6-bisaryl-2-pyridine-carboxamide.

Les documents US4916145, Jefferson et al, Journal of Medicinal Chemistry 1989, vo. 32, pages 1814 - 1820, Brenna et al, Journal of the Chemical Society, Perkin Transactions 1, 1998, pages 901 - 904 divulgue des composés spécifiques semblable des intermédiaires de formule IV.

Les composés selon la présente invention répondent à la formule (IV) : 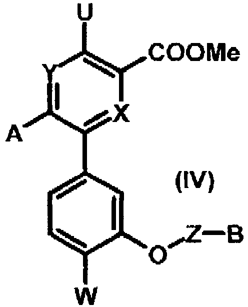 dans laquelle :
X et Y représentent indépendamment l'un de l'autre un atome d'azote ou un chaînon -CR3-₁ où R3 représente un atome d'hydrogène ou d'halogène ou un groupe alkyle ou alcoxy ;
U représente un atome d'hydrogène ou un groupe NHR7 où R7 est un atome d'hydrogène ou un groupe alkyle ;
A représente un groupe aryle, hétéroaryle ou hétérocycloalkyle éventuellement substitué ;
W représente un atome d'halogène, un groupe alkyle ou un groupe halogénoalkyle ;
Z représente une liaison, un groupe cycloalkylène ou un groupe alkylene éventuellement substitué par un ou plusieurs groupes choisis parmi un atome d'halogène et les groupes alkyle, hydroxy et alcoxy ;
B représente :
   - soit un groupe -NR4R5, où R4 et R5 représentent indépendamment l'un de l'autre un groupe alkyle, cycloalkyle, hydroxyalkyle ou fluoroalkyle, ou bien R4 et R5 forment, avec l'atome d'azote auquel ils sont rattachés, un cycle à 5 ou 6 chaînons, tel qu'un cycle pyrrolidinyle ou pipéridinyle, éventuellement substitué par un groupe alkyle,
   - soit un hétérocycle de formule suivante : 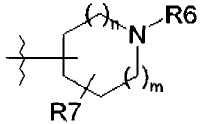
où m et n représentent indépendamment l'un de l'autre 0, 1 ou 2, et où R6 et R7 représentent indépendamment l'un de l'autre un atome d'hydrogène ou un groupe alkyle ou cycloalkyle ;
leurs sels, leurs énantiomères et diastéréoisomères, y compris leurs mélanges racémiques.

Les composés de formule (IV) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (IV) peuvent exister à l'état de bases ou salifiés par des acides ou des bases, notamment des acides ou des bases pharmaceutiquement acceptables. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (IV), font également partie de l'invention.

Les composés de formule (IV) peuvent également exister sous forme d'hydrates ou de solvats, à savoir sous forme d'associations ou de combinaisons avec une ou plusieurs molécules d'eau ou avec d'un solvant. De tels hydrates et solvats font également partie de l'invention.

Parmi les composés de formule (IV) objets de l'invention, on peut citer un sous-groupe de composés qui se définit comme suit :
X et Y représentent indépendamment l'un de l'autre un atome d'azote ou un chaînon -CR3-, où R3 représente un atome d'hydrogène ou un groupe alcoxy ;
   et/ou
U représente un atome d'hydrogène ou un groupe NHR7 où R7 est un atome d'hydrogène ou un groupe alkyle ;
   et/ou
A représente un groupe aryle, hétéroaryle ou hétérocycloalkyle choisi parmi les groupes phényle, benzodioxolyle, thiényle, thiazolyle, pyridinyle, pyrazolyle et pyrrolidinone, ledit groupe aryle ou hétéroaryle étant éventuellement substitué en des positions quelconques par un ou plusieurs groupes choisis parmi un atome d'halogène et les groupes cyano, alkyle, halogénoalkyle, hydroxy, alcoxy, -O-(CH₂)ₚ-alkyle, halogénoalcoxy, -NRR', -NR-CO-alkyle et -SO₂-alkyle, où R et R' représentent indépendamment l'un de l'autre un atome d'hydrogène ou un groupe alkyle et p est un nombre entier compris entre 1 et 5 et plus particulièrement entre 1 et 3 ;
   et/ou
W représente un atome d'halogène, un groupe alkyle ou un groupe halogénoalkyle ;
   et/ou
Z représente une liaison ou un groupe alkylène éventuellement substitué par au moins un groupe choisi parmi un atome d'halogène et les groupes alkyle et hydroxy ;
   et/ou
B représente :
   - soit un groupe -NR4R5, où R4 et R5 représentent indépendamment l'un de l'autre un groupe alkyle, hydroxyalkyle, ou bien R4 et R5 forment, avec l'atome d'azote auquel ils sont rattachés, un cycle à 5 ou 6 chaînons, tel qu'un cycle pyrrolidinyle ou pipéridinyle,
   - soit un hétérocycle de formule suivante : 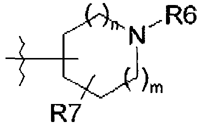
où m et n représentent indépendamment l'un de l'autre 0, 1 ou 2, et où R6 et R7 représentent indépendamment l'un de l'autre un atome d'hydrogène ou un groupe alkyle ou cycloalkyle.

Parmi les composés de formule (IV) objets de l'invention, on peut citer un deuxième sous-groupe de composés pour lesquels X et Y représentent indépendamment l'un de l'autre un atome d'azote ou un chaînon -CR3-, où R3 représente un atome d'hydrogène ou d'halogène ou un groupe alkyle ou alcoxy.

Plus particulièrement, parmi les composés de formule (IV) du deuxième sous-groupe, objets de l'invention, on peut citer un sous-groupe de composés pour lesquels lesquels X et Y représentent indépendamment l'un de l'autre un atome d'azote ou un chaînon -CR3-, où R3 représente un atome d'hydrogène ou un groupe alcoxy.

Parmi les composés de formule (IV) objets de l'invention, on peut citer un troisième sous-groupe de composés pour lesquels U représente un atome d'hydrogène ou un groupe NHR7 où R7 est un atome d'hydrogène ou un groupe alkyle.

Parmi les composés de formule (IV) objets de l'invention, on peut citer un quatrième sous-groupe de composés pour lesquels A représente un groupe aryle, hétéroaryle ou hétérocycloalkyle éventuellement substitué.

Plus particulièrement, parmi les composés de formule (IV) du quatrième sous-groupe, objets de l'invention, on peut citer un sous-groupe de composés pour lesquels A représente un groupe aryle, hétéroaryle ou hétérocycloalkyle choisi parmi les groupes phényle, benzodioxolyle, thiényle, thiazolyle, pyridinyle, pyrimidinyle, pyridazinyle, pyrazinyle, pyrazolyle et pyrrolidinone, ledit groupe aryle, hétéroaryle ou hétérocycloalkyle étant éventuellement substitué en des positions quelconques par un ou plusieurs groupes choisis parmi un atome d'halogène et les groupes cyano, alkyle, halogénoalkyle, hydroxy, alcoxy, -O-(CH₂)ₚ-O-alkyle, halogénoalcoxy, -NRR', -NR-CO-alkyle, -SO- et -SO₂-alkyle, où R et R' représentent indépendamment l'un de l'autre un atome d'hydrogène ou un groupe alkyle et p est un nombre entier compris entre 1 et 5.

Encore plus particulièrement, parmi les composés de formule (IV) du quatrième sous-groupe, objets de l'invention, on peut citer un sous-groupe de composés pour lesquels A représente un groupe choisi parmi les groupes phényle, benzodioxolyle, thiényle, thiazolyle, pyridinyle, pyrazolyle et pyrrolidinone, ledit groupe aryle ou hétéroaryle étant éventuellement substitué en des positions quelconques par un à trois groupes choisis parmi un atome d'halogène et les groupes cyano, alkyle, halogénoalkyle, hydroxy, alcoxy, -O-(CH₂)ₚ-O-alkyle, halogénoalcoxy, -NRR', -NR-CO-alkyle et -SO₂-alkyle, où R et R' représentent indépendamment l'un de l'autre un atome d'hydrogène ou un groupe alkyle et p est un nombre entier compris entre 1 et 3.

Parmi les composés de formule (IV) objets de l'invention, on peut citer un cinquième sous-groupe de composés pour lesquels W représente un atome d'halogène, un groupe alkyle ou un groupe halogénoalkyle.

Parmi les composés de formule (IV) objets de l'invention, on peut citer un sixième sous-groupe de composés pour lesquels Z représente une liaison, un groupe cycloalkylène ou un groupe alkylène éventuellement substitué par 1 ou 2 groupes choisis parmi un atome d'halogène et les groupes alkyle, hydroxy et alcoxy.

Plus particulièrement, parmi les composés de formule (IV) du sixième sous-groupe, objets de l'invention, on peut citer un sous-groupe de composés pour lesquels Z représente une liaison ou un groupe alkylène éventuellement substitué par au moins un groupe choisi parmi un atome d'halogène et les groupes alkyle et hydroxy.

Parmi les composés de formule (IV) objets de l'invention, on peut citer un septième sous-groupe de composés pour lesquels B représente :
- soit un groupe -NR4R5, où R4 et R5 représentent indépendamment l'un de l'autre un groupe alkyle, cycloalkyle, hydroxyalkyle ou fluoroalkyle, ou bien R4 et R5 forment, avec l'atome d'azote auquel ils sont rattachés, un cycle à 5 ou 6 chaînons, tel qu'un cycle pyrrolidinyle ou pipéridinyle, éventuellement substitué par un groupe alkyle,
- soit un hétérocycle de formule suivants : 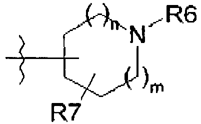
où m et n représentent indépendamment l'un de l'autre 0, 1 ou 2, et où R6 et R7 représentent indépendamment l'un de l'autre un atome d'hydrogène ou un groupe alkyle ou cycloalkyle.

Plus particulièrement, parmi les composés dé formule (IV) du septième sous-groupe, objets de l'invention, on peut citer un sous-groupe de composés pour lesquels B représente un groupe -NR4R5, où R4 et R5 représentent indépendamment l'un de l'autre un groupe alkyle, cycloalkyle, hydroxyalkyle ou fluoroalkyle, ou bien R4 et R5 forment, avec l'atome d'azote auquel ils sont rattachés, un cycle à 5 ou 6 chaînons, tel qu'un cycle pyrrolidinyle ou pipéridinyle, éventuellement substitué par un groupe alkyle.

Encore plus particulièrement, parmi les composés de formule (IV) du septième sous-groupe, objets de l'invention, on peut citer un sous-groupe de composés pour lesquels B représente un groupe -NR4R5, où R4 et R5 représentent indépendamment l'un de l'autre un groupe alkyle, hydroxyalkyle ou fluoroalkyle, ou bien R4 et R5 forment, avec l'atome d'azote auquel ils sont rattachés, un cycle à 5 ou 6 chaînons, tel qu'un cycle pyrrolidinyle ou pipéridinyle.

Plus particulièrement, parmi les composés de formule (IV) du septième sous-groupe, objets de l'invention, on peut citer un sous-groupe de composés pour lesquels B représente un hétérocycle de formule suivante : 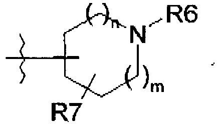 où m et n représentent indépendamment l'un de l'autre 0, 1 ou 2, et où R6 et R7 représentent indépendamment l'un de l'autre un atome d'hydrogène ou un groupe alkyle ou cycloalkyle.

Dans le cadre de la présente invention, et sauf mention différente dans le texte, on entend par :
- un atome d'halogène : un atome de fluor, de chlore, de brome ou d'iode ;
- un groupe alkyle : un groupe aliphatique saturé linéaire, comprenant de 1 à 5 atomes de carbone ou quand la chaîne alkyle comprend au moins trois atomes de carbone pouvant être linéaire, ramifié ou partiellement cyclisé. A titre d'exemples, on peut citer les groupes méthyle, éthyle, propyle, isopropyle, butyle, isobutyle, tertbutyle, pentyle, méthylène-cyclopropyle ;
- un groupe alkylène : un groupe alkyle tel que défini ci-dessus, qui est divalent. A titre d'exemple, on peut citer un groupe diméthylène (-CH₂-CH₂-), propylène, butylène, éthylène, ou 2-méthylpropylène ;
- un groupe cycloalkyle : un groupe cyclique saturé, qui comprend de 3 à 8 atomes de carbone et qui est cyclique. A titre d'exemples, on peut citer les groupes cyclopropyle, cyclopentyle, cyclohexyle et cycloheptyle ;
- un groupe hétérocycloalkyle : un groupe cycloalkyle tel que défini ci-dessus, dont un ou deux carbones ont été substitués par un atome d'azote. A titre d'exemple, on peut citer le groupe pyrrolidinone et le groupe pipéridine;
- un groupe aryle : un groupe aromatique monocyclique comprenant 5 ou 6 atomes de carbones, par exemple un groupe phényle ;
- un groupe hétéroaryle : un groupe aromatique cyclique comprenant 5 ou 6 atomes dont un ou plusieurs hétéroatomes tels que N et/ou S. A titre d'exemple de groupes hétéroaryles, on peut citer un groupe thiényle, thiazolyle, pyridinyte, pyrimidinyle, pyridazinyle, pyrazinyle, pyrazolyle ;
- un groupe cycloalkylène : un groupe cycloalkyle tel que défini ci-dessus qui est divalent ;
- un groupe fluoroalkyle : un groupe alkyle tel que défini ci-dessus, dont un ou plusieurs atomes d'hydrogène ont été substitués par un atome de fluor. A titre d'exemple, on peut citer le groupe trifluorométhyle ;
- un groupe alcoxy : un groupe de formule -O-alkyle où le groupe alkyle est tel que précédemment défini.

Parmi les composés décrits dans la présente invention, on peut citer un sous groupe de composés répondant à la formule (IV) dans laquelle :
X et Y représentent indépendamment l'un de l'autre un atome d'azote ou un groupe CH ;
A représente un groupe phényle, pyridinyle, ou pyrrolidinone, substitué en des positions quelconques par 1 à 2 groupes choisis parmi un atome d'halogène, tel que chlore ou fluor, et les groupes alkyle, trifluorométhyle, méthoxy et N,N-diméthylamine ;
U représente un atome d'hydrogène ou un groupe NHR7 où R7 est un atome d'hydrogène ;
W représente un atome de chlore ou un groupe trifluorométhyle ;
Z représente une liaison ou un groupe alkylène éventuellement substitué par un groupe méthyle;
B représente :
   - soit un groupe -NR4R5, où R4 et R5 représentent indépendamment l'un de l'autre un groupe alkyle ou bien R4 et R5 forment, avec l'atome d'azote auquel ils sont rattachés, un cycle à 5 ou 6 chaînons,
   - soit des hétérocycles de formule suivante: 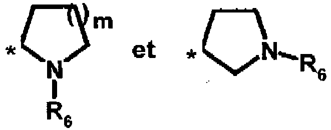
où m = 1 ou 2 et R6 représente un groupe éthyle ou méthyle.

On peut également citer un deuxième sous groupe de composés parmi les composés préférés et répondant à la formule (IV) dans laquelle :
X représente un atome d'azote et Y un groupe CH ;
A représente un groupe phényle ou pyridinyle, substitué dans les positions 2 ,4 ,5 et 6 par un ou deux groupes choisis parmi un atome d'halogène, tel que chlore ou fluor, et les groupes alkyles, tels que méthyle, éthyle ou isopropyle, trifluorométhyle, méthoxy et N,N-diméthylamine ;
U représente un atome d'hydrogène ou un groupe NHR7 où R7 est un atome d'hydrogène ;
W représente un atome de chlore ou un groupe trifluorométhyle ;
Z représente une liaison ou un groupe éthylène, propylène ou méthylpropylène ;
B représente :
   - soit un groupe -NR4R5, où R4 et R5 représentent indépendamment l'un de l'autre un groupe méthyle, éthyle ou propyle ou forment ensemble avec l'atome d'azote auquel ils sont rattachés un cycle à 5 ou 6 chaînons,
   - soit un hétérocycle de formule suwante :

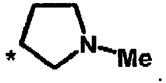

Conformément à l'invention, on peut préparer les composés de formule générale (IV) selon le procédé qui suit, illustré dans le schéma 1.

Quand, dans le composé de départ de formule (XI), X représente un atome d'azote, Y représente un atome de carbone (c'est-à-dire un groupe de formule -CR₃- telle que définie en rapport avec les composés de formule (I) selon l'invention) et U représente un atome d'hydrogène, ou bien X représente un atome de carbone, Y représente un atome d'azote et U représente un atome d'hydrogène, ou encore X et Y représentent un atome d'azote et U représente un atome d'hydrogène, alors on peut effectuer dans une étape (i) une réaction de couplage de type SUZUKI catalysée par un dérivé de palladium (0) tel que le tétrakis(triphénylphosphine)palladium (0) [Pd(PPh₃)₄] entre le composé de formule (XI) (où Q = OH ou Br et T = atome d'halogène, tel qu'un atome de brome ou d'iode) et un acide boronique de formule (IX), où GP représente un groupe benzyle éventuellement substitué par un ou plusieurs groupes alcoxy, en présence d'une base telle que le phosphate de potassium et dans un solvant tel que le N,N-diméthylformamide (DMF) à la température d'environ 95°C. Cette réaction permet de substituer régiosélectivement la fonction T par le noyau phénoxy pour conduire au composé (VIII).

La fonction OH du composé (VIII) est ensuite transformée en un groupe partant tel que trifluorométhanesulfonate (OTf), dans l'étape (ii), à l'aide d'anhydride trifluorométhanesulfonique en présence d'une base telle que la triéthylamine (TEA) et dans un solvant tel que le dichlorométhane (DCM) pour conduire au composé de formule (VII).

Le groupe trifluorométhanesulfonate ainsi obtenu permet dans l'étape (iii), grâce à sa réactivité, d'introduire le noyau A par une réaction de couplage organopalladiée de type :
- soit SUZUKI entre le composé (VII) et un acide ou un ester boronique de formules respectives A-B(OH)₂ ou 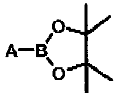 en présence d'une quantité catalytique de dérivé du palladium tel que le Pd(PPh₃)₄, en présence d'une base telle que le phosphate de potassium et d'un solvant tel que le DMF, à la température de 90°C ;
- soit STILLE entre le composé (VII) et un dérivé aryltributylstannane ou hétéroarylstannane ASnBu₃ en présence d'une quantité catalytique d'iodure de cuivre (Cul) et d'un dérivé de palladium (II) tel que le dichlorure de [1,1'-bis(cyclopentadiènyldiphénylphosphino)ferrocène] palladium (II) [PdCl₂(dppf)] et d'un solvant tel que le 1,4-dioxanne à la température de 90°C ;
- soit HARTWIG-BUCHWALD entre le composé (VII) et un amide tel que la 2-pyrrolidinone, en présence d'une quantité catalytique d'une phosphine telle que le 9,9-diméthyl-4,5-bis-(diphénylphosphino)xanthène (XantPhos) et d'un dérivé du palladium (0) tel que le tris(dibenzylidèneacétone)dipalladium (0) [Pd₂(dba)₃], en utilisant une base telle que le carbonate de césium, et dans un solvant tel que le DMF à la température de 70°C.

On obtient ainsi le composé (VI).

Alternativement, dans le composé de départ de formule (XI), quand X et Y représentent des atomes de carbone et U représente un atome d'hydrogène, Q un atome d'iode et T un atome de brome, ou bien X et Y représentent un atome d'azote et U représente un groupe NHR (tel que défini en rapport avec les composés de formule (1) selon l'invention), Q et T des atomes de chlore, de brome ou d'iode on inverse l'ordre d'introduction des groupes A et phénoxy sur le noyau phényle de départ (schéma 1 ).

Dans ce cas, une première réaction de type SUZUKI (étape (iv)) avec un acide boronique A-B(OH)₂ permet d'introduire sélectivement un groupe A à la place de l'atome d'halogène Q. La réaction est effectuée en présence d'une quantité catalytique d'un dérivé du palladium tel que le Pd(PPh₃)₄ et d'une base telle que le carbonate de césium, dans un solvant tel que le DMF à la température de 90°C. On réalise ensuite une deuxième réaction de type SUZUKI (étape (v)) entre l'ester boronique (XII) ou l'acide boronique (IX) et le composé (X), en présence d'une quantité catalytique d'un dérivé du palladium tel que le Pd(PPh₃)₄ et d'une base telle que le carbonate de césium, dans un solvant tel que le DMF à la température de 90°C. On obtient ainsi le composé (VI).

La déprotection de la fonction phénol du composé de formule (VI) par le tribromure de bore à -78°C, l'acide trifluoroacétique (TFA) à température ambiante ou le chlorure d'hydrogène à 0°C dans le DCM (étape (vi)) conduit au composé de formule (V).

L'introduction du groupement Z-B dans l'étape (vii) peut être effectuée :
- soit par alkylation du composé (V) avec un dérivé chloré CI-Z-B en présence d'une base minérale faible telle que le carbonate de césium et dans un solvant polaire aprotique tel que le DMF à une température comprise entre 80 et 100°C, telle que 90°C,
- soit par réaction de MITSUNOBU entre le composé (V) et un alcool de formule HO-Z-B en présence de triphénylphosphine, d'azodicarboxylate de diisopropyle (DIAD), et d'une quantité catalytique de base organique faible telle que la TEA à 0°C dans un solvant aprotique tel que le tétrahydrofurane (THF).

Le composé de formule (IV) est ensuite saponifié dans l'étape (viii), à l'aide d'une base minérale forte telle que l'hydroxyde de potassium dans un mélange eau/méthanol maintenu à température ambiante (T.A.) ou chauffé à reflux, pour conduire, après acidification avec un acide fort tel que l'acide chlorhydrique (HCl) 1 N, au composé (III).

Dans le cadre de la présente invention par température ambiante, on entend une température comprise entre 20 et 25°C.

Une réaction de couplage peptidique (étape (ix)) entre le composé (III) et des amines de formule (II) en présence d'un agent de couplage tel que le carbonyldiimidazole (CDI), le tétrafluoroborate de N-[(1H-benzotriazol-1-yloxy)(diméthylamino)méthylidène]-N-méthylméthanaminium (TBTU) ou le chlorhydrate de N-[3-(diméthylamino)propyl-N'-éthyl carbodiimide (EDC .HCl) et d'une base organique telle que la N,N',diisopropyléthylamine (DIEA) et dans un solvant polaire aprotique tel que le DMF à température ambiante conduit aux composés de formule (I) conformes à la présente invention.

Le schéma 2 décrit la synthèse des dérivés boroniques (IX) et (XII).

### Cas où W = CF₃

Le 2-amino-5-nitrophénol (XVI) est transformé en dérivé iodé (XV) par une réaction de SANDMEYER en milieu aqueux à 0°C, en présence d'iodure de sodium et avec un co-solvant tel que le diméthylsulfoxyde (DMSO). La protection de la fonction phénol (étape i) par un groupement benzyle est effectuée avec un dérivé halogéné tel que le bromure de benzyle en présence d'une base telle que le carbonate de potassium (K₂CO₃) dans un solvant tel que le DMF à 60°C. La substitution de l'atome d'iode par le groupe trifluorométhyle *(*Eur. J. Org. Chem. (2003) pp. 1559-1568*)*, réalisée avec le trifluorométhyltriméthylsilane en présence de Cul et de fluorure de potassium dans un solvant tel que la N-méthylpyrrolidinone (NMP) à 45°C (étape ii), conduit au composé (XIV) qui, après réduction de la fonction nitro en fonction amino par un réducteur tel que le fer à 70°C dans un mélange éthanol (EtOH)/eau/acide acétique (AcOH), est transformé en dérivé iodé (XIII) à l'aide d'une deuxième réaction de SANDMEYER (étape iii).

Dans l'étape (iv), une réaction d'échange métal-halogène effectuée entre le composé (XIII) et le chlorure d'isopropyle magnésium (iPrMgCl) à -50°C dans un solvant tel que le THF, suivie de l'adition de triisopropylborate, conduit après une acidolyse avec un acide tel que l'HCl 5N, à l'acide boronique (IX).

### Cas où W = Cl

La protection du 2-chloro-5-iodophénol (XVIIa) par un groupe benzyle ou para-méthoxybenzyle comme décrit à l'étape (i) conduit au dérivé iodé (XVII) qui peut-être transformé :
- soit, dans l'étape (iv), en acide boronique (IX) comme décrit précédemment,
- soit, dans l'étape (v), en ester boronique (XII) par une réaction de couplage avec le 4,4,4',4',5,5,5',5'-octaméthyl-2,2'-bi-dioxaborolane en présence d'une base telle que l'acétate de potassium (KOAc) et d'une quantité catalytique d'un dérivé du palladium (II) tel que le [PdCl₂(dppf)], dans un solvant tel que le DMSO à 100°C.

Alternativement, un sous-groupe de composés de formule (la) conforme à la présente invention où le groupe A représente un noyau 2,6-diméthoxybenzène a été préparé de la façon suivante (schéma 3) :
- La condensation du 2,6-diméthoxybenzaldéhyde sur l'acide 2-cyanoacétique (étape i) en présence d'acétate d'ammonium et d'une base telle que la pyridine en chauffant au reflux d'un solvant tel que le toluène conduit au dérivé (XX).
- Le dérivé (XX) est ensuite traité par de l'hydrure de diisobutylaluminium (DIBAL-H) à 0°C dans un solvant tel que le toluène. L'aldéhyde ainsi obtenu est mis en réaction (étape ii) avec le 2-azido acétate d'éthyle à -10°C en présence d'une base telle que l'éthylate de sodium dans un solvant tel que l'éthanol pour obtenir le diène (XIX). A l'étape (iii), le traitement du diène (XIX) par la triphénylphosphine à T.A. dans un solvant tel que le DCM conduit à l'azatriphénylphosphoranylidène (XVIII).
- La réaction (étape iv) entre le dérivé (XVIII) et l'aldéhyde (XVII) dans un solvant tel que l'acétonitrile conduit, via la cyclisation *in situ* à 100°C d'une imine intermédiaire selon une séquence cyclisation électrocyclique / déhydrogénation, au dérivé pyridinique (IVa).
- Les séquences d'alkylation ou de réaction de MITSUNOBU (étape v), de saponification (étape vi) puis de couplage peptidique (étape vii) décrites auparavant dans le schéma 1 et appliquées au dérivé (IVa) conduisent aux composés de formule (la) conformes à la présente invention.

Dans les schémas 1 et 2, les composés de départ, les intermédiaires et les réactifs, quand leur mode de préparation n'est pas décrit sont disponibles dans le commerce ou décrits dans la littérature, ou bien peuvent être préparées selon des méthodes qui sont connues de l'homme de métier.

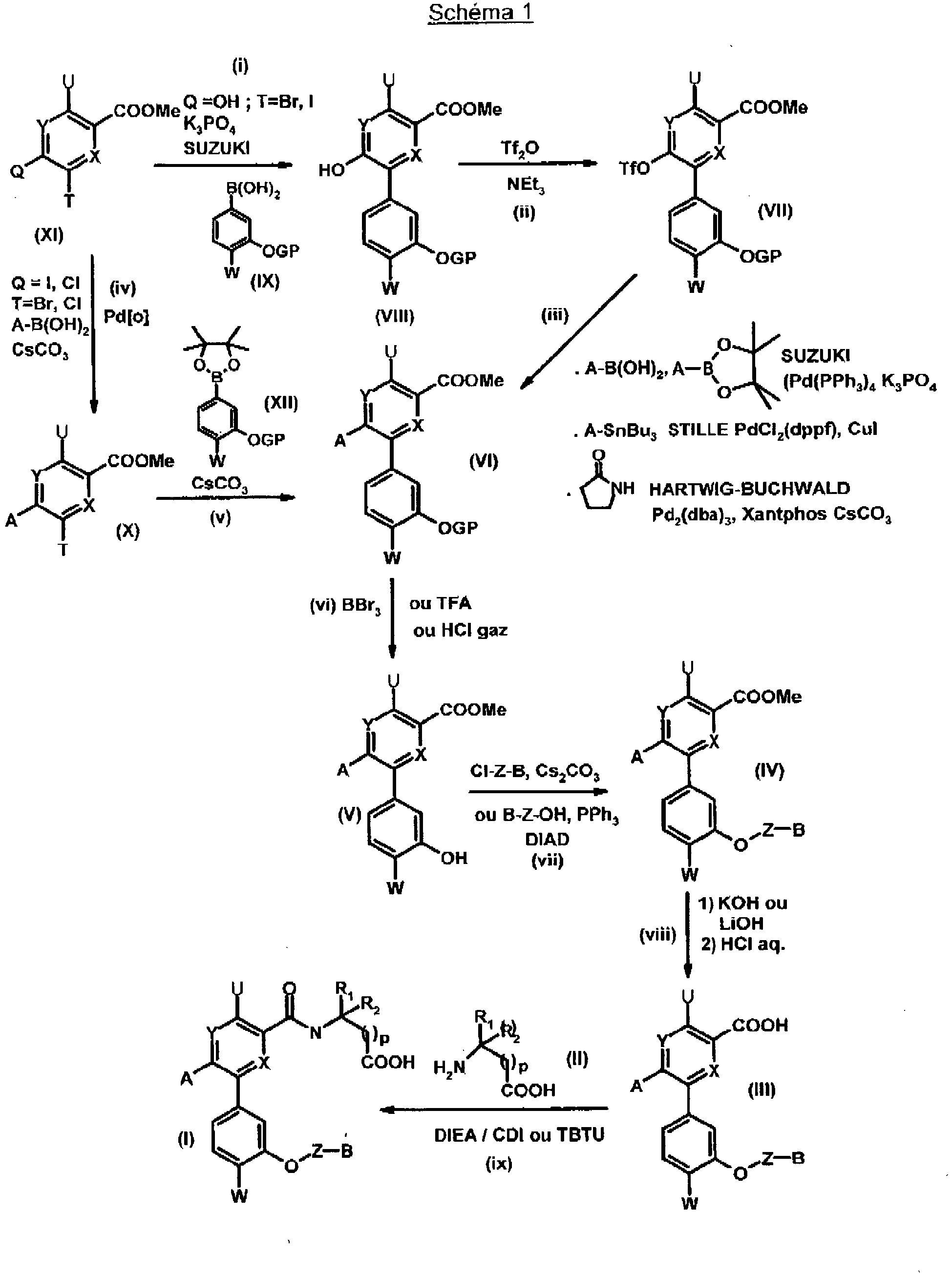

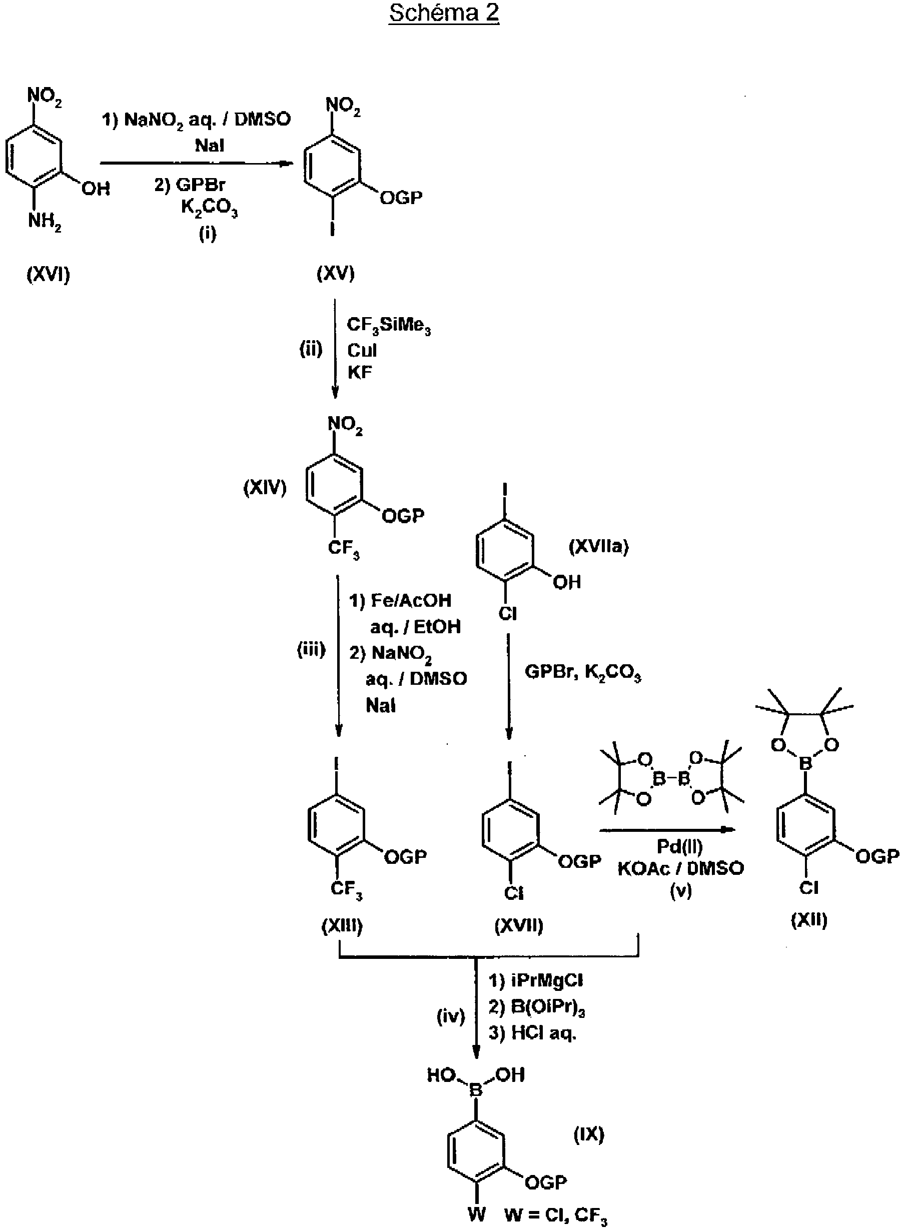

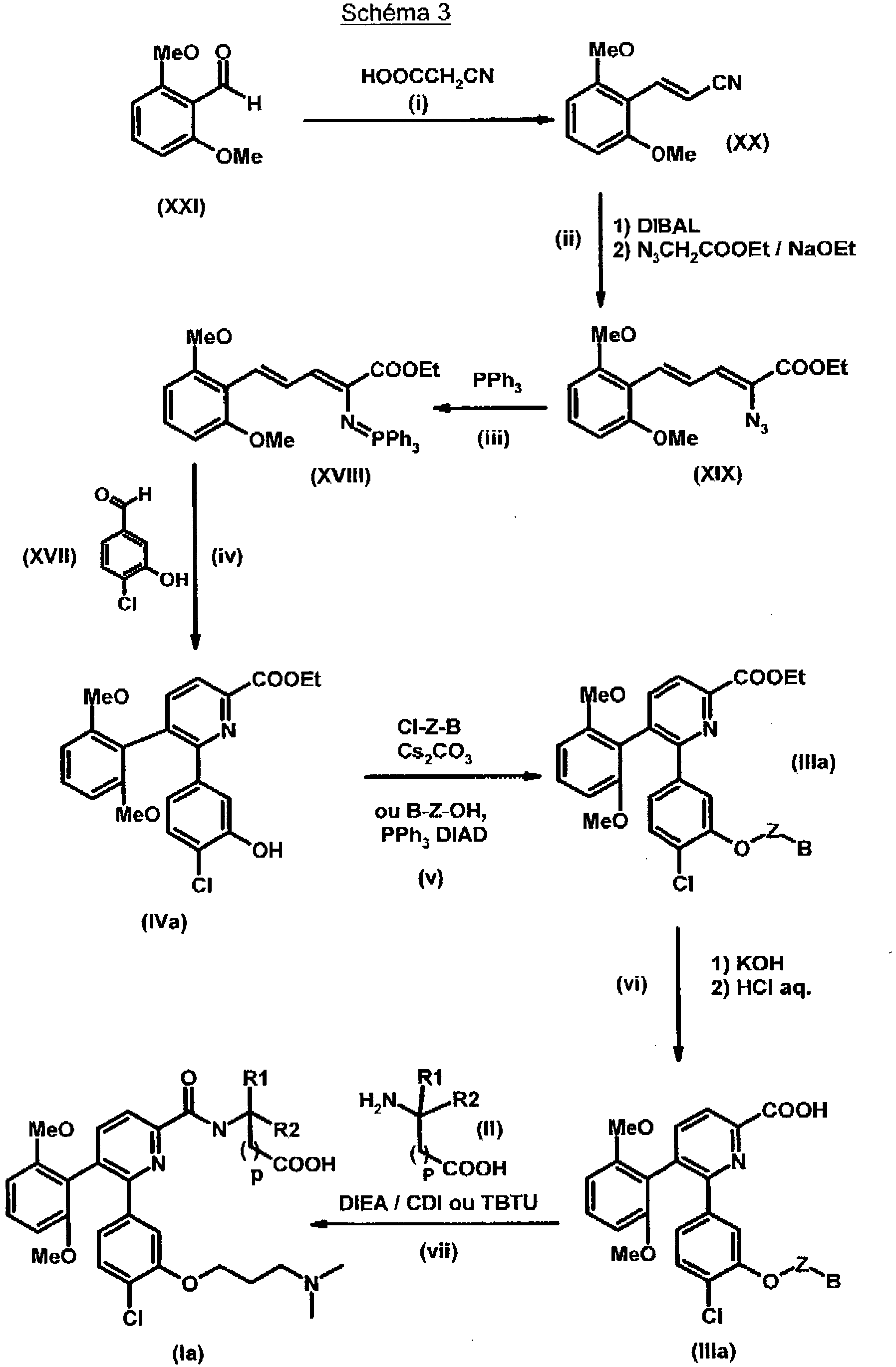

Les exemples suivants illustrent la préparation de certains composés conformes à l'invention. Les numéros des composés exemplifiés renvoient à ceux du tableau donné plus loin qui illustre les structures chimiques et les propriétés physiques de quelques composés selon l'invention.

Les abréviations suivantes sont utilisées :
- AcOEt: Acétate d'éthyle
- AcOH: Acide acétique
- BSA: N,O-Bis(trimëthyisilyl)acétamide
- CDI: Carbonyldiimidazole
- Cul: Iodure de cuivre
- DCM: Dichlorométhane
- DIBAL-H: Hydrure de diisobutylaluminium
- DIAD: Azodicarboxylate de diisopropyle
- DME: Diméthoxyéthane
- DMF: Diméthylformamide
- DMSO: Dimethyl sulfoxide
- EDC .HCl: Chlorhydrate de N-[3-(diméthylamino)propyl-N'-éthyl carbodiimide
- EtOH: Ethanol
- h: Heure(s)
- HCl: Acide chlorhydrique
- K₂CO₃: Carbonate de potassium
- KOAc: Acétate de potassium
- K₃PO₄: Phosphate de potassium ou tetraoxophosphate de tripotassium
- Na₂CO₃: Carbonate de sodium
- NH₄Cl: Chlorure d'ammonium
- NaHCO₃: Hydrogénocarbonate de sodium
- Na₂SO₄: Sulfate de sodium
- NMP: N-méthylpyrrolidinone
- PdCl₂ (dppf): Dichlorure de[1,1'-] bis(cyclopentadiènyldiphénylphosphino)ferrocène] palladium (II)
- Pd(PPh₃)₄: Tetrakis (triphénylphosphine) palladium (0)
- Pd₂(dba)₃: Tris(dibenzylidèneacétone)dipalladium (0)
- TBTU: Tétrafluoroborate de N-[(1H-benzotriazol-1-yloxy)(diméthylamino)méthylidène]-N-méthylméthanaminium.
- TEA: Triéthylamine
- TFA: Acide trifluoroacétique
- THF: Tétrahydrofurane
- T.A.: Température ambiante

### Exemple 1: Acide 1-({[6-{4-chloro-3-[3-(diméthylamino)propoxy]phényl}-5-(2,6-diméthoxyphényl)pyridin-2-yl]carbonyl}amino)cyclohexanecarboxylique (composé N°12)

### 1.1. (2E)-3-(2,6-diméthoxyphényl)acrylonitrile

A une solution de 61 g (367 mmoles) de 2,6-diméthoxybenzaldéhyde dans 360 mL de toluène sont ajoutés successivement 1,13 g (14,7 mmoles) d'acétate d'ammonium, 40,4 mL (500 mmoles) de pyridine et 31,2 g (367 mmoles) d'acide 2-cyanoacétique. Le milieu réactionnel est porté à reflux 15 h en distillant l'azéotrope toluène-eau dans un montage de Dean-Stark. La solution est reprise par 500 mL de toluène et 40 mL de pyridine et à nouveau portée à reflux pendant 48 h dans le montage de Dean-Stark. Après concentration sous pression réduite, le résidu est repris par 800 mL de dichlorométhane (DCM) et lavé successivement avec 1 L d'une solution aqueuse d'HCl 1 N, 500 mL d'une solution aqueuse saturée de carbonate de sodium (Na₂CO₃) et 1 L d'eau. Après séchage sur sulfate de sodium (Na₂SO₄) et concentration sous pression réduite, on obtient 62 g de (2E)-3-(2,6,diméthoxyphényl)acrylonitrile sous forme d'huile brune utilisée tel quel à l'étape suivante.
Rendement = 89 %

### 1.2. (2E)-3-(2,6-diméthoxyphényl)actylaldéhyde

A une solution de 61 g (322 mmoles) de (2E)-3-(2,6-diméthoxyphényl)acrylonitrile dans 600 mL de toluène anhydre placée sous argon et refroidie à 0°C sont ajoutés 355 mL d'une solution 1M d'hydrure de diisobutylaluminium (DIBAL-H) (355 mmoles) dans le toluène en maintenant la température à 0°C. Le milieu réactionnel est ensuite ramené à température ambiante (T.A.) et agité pendant 3 h. On ajoute alors 13 mL (322 mmoles) de méthanol puis goutte à goutte 170 g (174 mmoles) d'une solution aqueuse d'acide sulfurique à 10 % en poids. La suspension est agitée une heure puis filtrée sur gâteau de célite. Le filtrat est lavé avec 500 mL d'eau puis séché sur Na₂SO₄ et concentré sous pression réduite. Le résidu obtenu est repris par 500 mL de DCM et filtré sur colonne de silice en éluant avec du DCM. Après concentration sous pression réduite, on obtient 37 g de (2E)-3-(2,6-diméthoxyphényl)acrylaldéhyde sous forme d'huile incolore.
Rendement = 60 %.

### 13. (2E,4E)-2-azido-5-(2,6-diméthoxyphénvl)penta-2,4-diénoate d'éthyle

A une solution de 15,5 g (673 mmoles) de sodium dans 500 mL d'éthanol EtOH absolu refroidie à -10°C et placée sous argon, est ajoutée goutte à goutte sous agitation une solution de 37 g (192 mmoles) de (2E)-3-(2,6-diméthoxyphényl)acrylaldéhyde et 85,7 g (664 moles) de 2-azidoacétate d'éthyle dans 200 mL d'EtOH absolu en maintenant la température à -10°C. Après 3h d'agitation à -10°C, le milieu réactionnel est ensuite agité pendant 15 h à T.A. puis versé sur 600 mL d'une solution aqueuse de chlorure d'ammonium (NH₄Cl) à 30% en poids. Après filtration sur un fritté et rinçage avec 2 x 200 mL d'eau, le précipité est repris par 600 mL de DCM, séché sur Na₂SO₄ et concentré sous pression réduite, ce qui conduit à 42 g de (2E,4E)-2-azido-5-(2,6-diméthoxyphényl)penta-2,4-diénoate d'éthyle sous forme de solide jaune.
Rendement = 80 %
F(°C) = 138.

### 1.4 (2E, 4E)- 5-(2,6-diméthoxyphényl)-2-[(triphénylphosphoranylidène)amino]penta-2,4-diénoate d'éthyle

A une solution de 4,37 g (16,7 mmoles) de triphénylphosphine dans 40 mL de DCM est ajoutée goutte à goutte une solution de 5 g de (2E,4E)-2-azido-5-(2,6-diméthoxyphényl)penta-2,4-diénoate d'éthyle (16,5 mmoles) dans 60 mL de DCM. Le milieu réactionnel est agité 2 h à T.A. puis concentré sous pression réduite. Le résidu obtenu est concrétisé dans 100 mL d'éther isopropylique puis filtré sur un fritté et rincé avec 50 mL d'éther isopropylique, ce qui permet d'obtenir 7,9 g de (2E,4E)-5-(2,6-diméthoxyphényl)-2-[(triphénylphosphoranylidène)amino]penta-2,4-diénoate d'éthyle sous forme de solide blanc.
Rendement = 91 %
F(°C) = 172

### 1.5. 6-(4-chloro-3-hydroxyphényl)-5-(2,6-diméthoxyphényl)pyridine-2-carboxylate d'éthyle

Une solution de 7,54 g (14 mmoles) de (2E,4E)-5-(2,6-diméthoxyphényl)-2-[(triphénylphosphoranylidène)amino]penta-2,4-diénoate d'éthyle et de 2,42 9 (15,4 mmoles) de 4-chloro-3-hydroxybenzaldéhyde dans 280 mL d'acétonitrile anhydre est portée à reflux durant 96 h. Après refroidissement à T.A., le milieu réactionnel est concentré sous pression réduite et le résidu obtenu est purifié par chromatographie sur colonne de gel de silice en éluant avec un gradient cyclohexane / acétate d'éthyle (AcOEt) de 0 à 30 % d'AcOEt. Après concentration sous pression réduite, on obtient 2,4 g de 6-(4-chloro-3-hydroxyphényl)-5-(2,6-diméthoxyphényl)pyridine-2-carboxylate d'éthyle sous forme de poudre blanche.
Rendement = 41 %
F(°C) = 182

### 1.6. 6-{4-chloro-3-[3-(diméthylamino)propoxy]phényl}-5-(2,6-diméthoxyphényl)pyridine-2-carboxylate d'éthyle

A une solution de 0,5 g (1,21 mmoles) de 6-(4-chloro-3-hydroxyphényl)-5-(2,6-diméthoxyphényl)pyridine-2-carboxylate d'éthyle dans 20 mL de DMF anhydre placée sous argon sont ajoutés 0,257 g (2,11 mmoles) de chlorhydrate de 3-chloro-N,N-diméthylpropane-1-amine et 3,40 g (1,34 mmoles) de carbonate de césium. Le milieu réactionnel est agité 15 min à T.A puis porté 2 h à 80°C. Après refroidissement à T.A., on ajoute 1 mL d'une solution aqueuse à 5 % d'acide citrique et l'ensemble est concentré sous pression réduite. Le résidu est repris par 50 mL d'AcOEt et lavé avec 10 mL d'une solution à 5 % de Na₂CO₃ puis 10 mL d'eau. Après séchage sur Na₂SO₄ et concentration sous pression réduite, le résidu est purifié par chromatographie sur une colonne de gel de silice, en éluant avec un gradient DCM / méthanol de 1 à 15 % de méthanol. Après concentration sous pression réduite, on obtient 0,55 g de 6-(4-chloro-3-[3-(diméthylamino)propoxy]phényl}-5-(2,6-diméthoxyphényl)pyridine-2-carboxylate d'éthyle sous forme d'huile.
Rendement = 91 %

### 1.7. Acide 6-{4-chloro-3-[3-(diméthylamino)propoxy]phényl}-5-(2,6-dimétroxyphényl)pyridine-2-carboxylique

A une solution de 5,63 g (11,28 mmoles) de 6-{4-chloro-3-[3-(diméthylamino)propoxy]phényl}-5-(2,6-diméthoxyphényl)pyridine-2-carboxylate d'éthyle dans 132 mL d'EtOH, sont ajoutés 3,16 g (56,38 mmoles) d'hydroxyde de potassium. Le milieu réactionnel est porté 2 h à reflux puis concentré sous pression réduite. Le résidu obtenu est repris par 10 mL d'eau puis neutralisé avec 56,5 mL (56,5 mmoles) d'une solution aqueuse d'HCl 1N. Le précipité obtenu est filtré sur un fritté et rincé avec 2 x 10 mL d'eau. Après séchage sous pression réduite, on obtient 5,25 g d'acide 6-{4-chloro-3-[3-(diméthylamino)propoxy]phényl}-5-(2,6-diméthoxyphényl)pyridine-2-carboxylique sous forme de poudre blanche.
Rendement = 100 %
F(°C) = 215

### 1.8. Acide 1-({[6-{4-chloro-3-[3-(diméthylamino)propoxy]phényl}-5-(2,6-diméthoxyphènyl)pyridin)-2-yl]carbonyl}amino)cyclohexanecarboxylique

A une solution de 200 mg (0,42 mmole) d'acide 6-{4-chloro-3-[3-(diméthylamino)propoxy]phényl}-5-(2,6-diméthoxyphényl)pyridine-2-carboxylique dans 50 mL de DMF anhydre sont ajoutés sous argon 0,74 mL (0,41 mmole) de diisopropyléthylamine et 409 mg (1,28 mmoles) de TBTU. Parallèlement, on porte à 90°C sous agitation et sous argon un mélange de 84 mg (0,59 mmole) d'acide 2-aminocyclohexane-2-carboxylique et de 0,18 mL (0,76 mmole) de N,O-Bis {triméthylsilyl} acétamide (BSA) dans 5 mL d'acéyonitrile anhydre. Après 2 h, le milieu se solubilise complètement et la solution est ramenée à T.A. puis ajoutée à la solution d'acide 6-{4-chloro-3-[3-(diméthylamino)propoxy]phényl}-5-(2,6-diméthoxyphényl)pyridine-2 carboxylique activée avec du TBTU. Après 18 h d'agitation à T.A., on ajoute 10 mL d'une solution aqueuse 0,5 N d'HCl et l'agitation est maintenue 3 h. Le milieu réactionnel est alors réparti dans un mélange de 20 mL d'AcOEt/éther 1:1 et 10 mL d'eau. Après extraction, la phase aqueuse est à nouveau extraite avec 10 mL d'un mélange éther/AcOEt 1:1. Les phases organiques sont réunies, lavées avec 2 x 10 mL d'eau, séchées sur Na₂SC₄ et concentrée sous pression réduite. Le résidu est ensuite purifié par HPLC sur phase inverse (RP18) en éluant avec un gradient HCl 0,01N / acétonitrile de 5 % à 100 % d'acétonitrile. Après concentration sous pression réduite et une lyophilisation, on obtient 106 mg de chlorhydrate d'acide 1-({[6-{4-chloro-3-[3-(diméthylamino)propoxy]phényl}-5-(2,6-diméthoxyphényl)pyridin-2-yl]carbonyl}amino)cyclohexanecarboxylique sous forme de poudre blanche.
Rendement = 41 %
F(°C) > 200.
M = C₃₂H₃₈ClN₃O₆ = 595 ; M+H = 596
RMN ¹H (ppm, d6-DMSO, 400 MHz) : 8,70 (s, 1H) ; 7,80 (d, 1H) 7,65 (d, 1H) 7,15 (m, 2H) ; 7,05 (d, 1H) ; 6,60 (d, 1H) ; 6,50 (d, 2H) ; 3,85 (t, 2H) ; 3,35 (s, 6H) ; 2,75 (m, 2H) ; 2,45 (s, 6H) ; 2,15 (m, 2H) ; 1,90 (m, 2H) 1,55 (m, 4H) ; 1,25 (m, 4H).

### Exemple 2 : Chlorhydrate d'acide 9-({[6-{4-chloro-3-[3-(diméthylamino)propoxy]phényl}-5-(2,6-diméthoxyphényl)pyridin-2-yl]carbonyl}amino)bicyclo[3.3.1]nonane-9-carboxylique (composé N°25)

### 2.1. 9-aminobicyclo[3.3.1.]nonane-9-carbonitrile

A une solution de 3 g (21,7 mmoles) de bicyclo[3.3.1.]nonane-9-one dans 40 mL d'EtOH, sont ajoutés successivement 21 mL d'eau, 1,12 g (22,8 mmoles) de cyanure de sodium, 4,52 mL (54,27 mmoles) d'une solution aqueuse d'ammoniaque 12N et 2,32 g (43,41 mmoles) de NH₄Cl. Le mélange réactionnel est agité 18 h à 50°C. On ajoute alors 5 mL d'une solution aqueuse d'ammoniaque 12N et le mélange est à nouveau chauffé 4 h à 50°C. La solution est refroidie à T.A. puis répartie dans 100 mL d'un mélange éther/solution aqueuse de soude 1 N 1:1. Après extraction, la phase organique est lavée avec 3 x 50 mL d'eau, séchée sur Na₂SO₄ et concentrée sous pression réduite. Le résidu obtenu est repris par 50 mL d'éther puis traité une minute avec un léger courant de chlorure d'hydrogène. Le chlorhydrate ainsi obtenu, est filtré, lavé avec 20 mL d'éther et réparti dans 100 mL d'un mélange DCM/solution aqueuse saturée de Na₂CO₃ 1:1. La phase aqueuse est à nouveau extraite avec 50 mL de DCM, les phases organiques étant ensuite réunies, lavées avec 2 x 20 mL d'eau et séchées sur Na₂SO₄. Après concentration sous pression réduite, on obtient 1,88 g de 9-aminobicyclo[3.3.1.]nonane-9-carbonitrile sous forme d'huile.
Rendement = 52 %

### 2.2. N-(9-cyanobicyclo[3.3.1]non-9-yl)benzamide

A une solution de 1,88 g (11,45 mmoles) de 9-aminobicyclo[3.3.1]nonan-9-carbonitrile dans 20 mL de THF sont ajoutés une solution de 2,37 g (17,17 mmoles) de carbonate de potassium dans 30 mL d'eau puis 1,37 mL (11,8 mmoles) de chlorure de benzoyle. Le milieu réactionnel est agité 1 h à T.A. puis réparti dans 100 mL d'un mélange DCM/eau 1:1. La phase organique est lavée avec 50 mL d'eau, séchée sur Na₂SO₄ et concentrée sous pression réduite. Le résidu obtenu est concrétisé dans 100 mL de pentane ce qui permet d'obtenir, après filtration et lavage avec du pentane, 2,81 g de N-(9-cyanobicyclo[3.3.1]non-9-yl)benzamide sous forme de poudre blanche.
Rendement = 92 %
F(°C) > 200

### 2.3. Acide 9-aminobicyclo[3.3.1]nonane-9-carboxylique

A une solution de 2,8 g (10,43 mmoles) de N-(9-cyanobicyclo[3.3.1]non-9-yl)benzamide dans 80 mL de THF sont ajoutés 200 mL d'une solution aqueuse d'HCl 12N La solution est agitée 20 h à T.A. et un précipité blanc apparaît progressivement. Après filtration du précipité sur fritté et rinçage avec 3 x 200 mL d'eau, on obtient 3,6 g d'acide 9-benzoylamino bicyclo[3.3.1]nonane-9-carboxylique humide.
3 g (10,44 mmoles) d'acide 9-benzoylamino bicyclo[3.3.1]nonane-9-carboxylique sont dissous dans 200 mL d'AcOH et 50 mL d'une solution aqueuse d'HCl 6N et le mélange est porté 18 h à reflux puis concentré partiellement par distillation de 150 mL de solvant. Après refroidissement, le mélange réactionnel est filtré et réparti dans 150 mL d'un mélange d'une solution aqueuse d'HCl 1N /éther 1:2. Après extraction, la phase aqueuse est concentrée puis traitée goutte à goutte avec une solution aqueuse de soude (NaOH) 12N pour amener le pH à 5-6. L'aminoacide ainsi précipité est filtré, lavé avec 3 x 50 mL d'eau et séché sous pression réduite ce qui conduit à 1,88 g d'acide 9-aminobicyclo[3.3.1]nonan-9-carboxylique sous forme de cristaux blancs.
Rendement = 98 %
F°(C) > 250.

### 2.4. Chlorhydrate d'acide 9-({[6-(4-chloro-3-[3-(diméthylamino)propoxylphényl}-5-(2,6-diméthoxyphényl)pyridin-2-yl]carbonyl}amino)bicyclo[3.3.1]nonane-9-carboxylique

Selon le procédé décrit dans l'exemple 18, on obtient, à partir de 400 mg (0,85 mmoles) d'acide 6-{4-chloro-3-[3-(diméthylamino)propoxylphényl}-5-(2,6-diméthoxyphényl)pyridine-2-carboxylique et 218 mg (1,19 mmoles) d'acide 9-aminobicyclo[3.3.1]nonane-9-carboxylique et après purification sur phase inverse et une lyophilisation. 220 mg de chlorhydrate d'acide 9-({[6-{4-chloro-3-{3-(diméthylamino)propoxy]phényl}-5-(2,6-diméthoxyphényl)pyridin-2-yl]carbonyl}amino)bicyclo[3.3.1]nonane-9-carboxylique sous forme de poudre blanche.
Rendement = 41 %
F(°C) : 197
M - C₃₅H₄₂ClN₃O₆ = 635 ; M+H = 636
RMN 1H (ppm, d6-DMSO, 400MHz) 8,45 (s, 1H) ;8,00 (d, 1H) ; 7,80 (d, 1H) ; 7,30 (t, 2H) ; 7,25 (s, 1H) ; 7,05 (d, 1H) ; 6,95 (dd, 1H) ; 6,70 (d, 1 H) ; 3,85 (t, 2H) ; 3,50 (s, 6H) ; 3,05 (m, 2H) ; 2,70 (s, 6H) ; 2,2-1,4 (m, 16H).

### Exemple 3 : Chlorhydrate d'acide 2-({[6-{4-chloro-3-[(1-méthylpyrrolidin-3-yl)oxy]phényl}-5-(2,6-diméthoxyphényl)pyridin-2-yl]carbonyl}amino)adamantane-2-carboxylique (composé N°15)

### 3.1. 6-{4-chloro-3-[(1-méthylpyrrolidin-3-yl)oxy]phényl}-5-(2,6-diméthoxyphényl)pyridine-2-carboxylate d'éthyle

A une solution de 300 mg (0,72 mmole) de 6-(4-chloro-3-hydroxyphenyl)-5-(2,6-dimethoxyphenyl)pyridine-2-carboxylate d'éthyle dans 2,5 mL de THF anhydre refroidie à 0°C et placée sous argon, sont ajoutés 88 mg (0,87 mmole) de 1-méthyl-3-hydroxypyrolidine, 246 mg (1,09 mmoles) de triphénylphosphine et 0,01 mL (0,07 mmoles) de TEA. Après solubilisation, on ajoute goutte à goutte à 0°C une solution de 0,24 mL (1,09 mmoles) de DIAD dans 2,5 mL de THF anhydre. Le milieu réactionnel est ramené à T.A., agité pendant 18 h puis repris par 50 mL d'AcOEt. La phase organique est lavée successivement avec 20 mL d'une solution aqueuse saturée de bicarbonate de sodium (NaHCO₃) puis 20 mL d'eau. Après séchage sur Na₂SO₄ et concentration sous pression réduite, le résidu obtenu est purifié par chromatographie sur une colonne de gel de silice en éluant avec un gradient DCM / méthanol de 0 à 20 % en méthanol. Après concentration sous pression réduite, on obtient 250 mg de 6-{4-chloro-3-[(1-méthylpyrrolidin-3-yl)oxy]phényl}-5-(2,6-diméthoxyphényl)pyridine-2-carboxylate d'éthyle sous forme d'huile.
Rendement = 70 %

### 3.2. Chlorhydrate d'acide 2-({[6-{4-chloro-3-[(1-méthylpyrrolidin-3-yl)oxy]phényl}-5-(2,6-diméthoxyphényl)pyridin-2-yl]carbonyl}amino)adamantane-2-carboxylique

Selon la séquence saponification/couplage peptidique décrite dans les exemples 1.7 et 1.8 respectivement, on obtient, à partir de 250 mg (0,5 mmole) de 6-{4-chloro-3-[(1-méthylpyrrolidin-3-yl)oxy]phényl}-5-{2,6-diméthoxyphényl)pyridine-2-carboxylate d'éthyle et de 100 mg (0,51 mmole) d'acide 2-aminoadamantane-2-carboxylique, 160 mg d'acide 2-({[6-{4-chloro-3-[2-(diméthylamino)éthoxy]phényl}-5-(2,6-diméthoxyphényl)pyridin-2-yl]carbonyl}amino)adamantane-2-carboxylique sous forme de poudre blanche.
Rendement= 47 %
F(°C) = 215
M = Ca₃₆H₄₀ClN₃O₆ = 645 ; M+H = 646
RMN¹H (ppm, d6-DMSO,40MHz) : 8,40 (s, 1H) ; 8,00 (d, 1H) ; 7,85 (d, 1 H) ; 7,35 (m, 2H) ; 7,05 (dd, 1H) ; 6,95 (t, 1H) ; 6,65 (d, 2H) ; 4,90 (m, 1H) ; 3,80 (m, 2H) ; 3,50 (s, 6H) ; 3,15 (m, 2H) ; 2,85 (m, 3H) ; 2,55 (s, 2H) ; 2,2-1,6 (m, 14H).

### Exemple 4 : Chlorhydrate d'acide 2-({[6-{4-chloro-3-[3-(diméthylamino)propoxy]phényl}-5-(2-méthylphényl)pyridin-2-yl]carbonyl}amino)adamantane-2-carboxylique (composé N° 18)

La synthèse du 6-bromo-5-hydroxy-2-pyridine carboxylate de méthyle est réalisée selon un procédé déjà décrit dans la littérature (J. Org. Chem., 1996, 4623-4633).

### 4.1. 2-(benzyloxy)-1-chloro-4-iodobenzène

Une suspension de 300 g (1179 mmoles) de 2-chloro-5-iodophénol et de 140 mL (1179 mmoles) de bromure de benzyle et de 195,5 g (1415 mmoles) de carbonate de potassium anhydre dans 1,2 L de DMF anhydre est agitée 5 h à 70°C puis ramenée à T.A.. Le milieu réactionnel est ensuite réparti dans 3 L d'un mélange éther/eau 2:1. La phase organique est lavée avec 2 x 1 L d'eau, séchée sur Na₂SO₄ et concentrée sous pression réduite et le résidu obtenu est concrétisé dans du pentane. On obtient ainsi 376 g de 2-(benzyloxy)-1-chloro-4-iodobenzène sous forme de poudre beige.
Rendement = 92 %
F(°C) = 72.

### 4.2. Acide [3-(benzyloxy)-4-chlorophényl]boronique

A une solution de 198 g (575 mmoles) de 2-(benzyloxy)-1-chloro-4-iodobenzène dans 1,2 L de THF anhydre placée sous argon et agitée à -50°C, sont ajoutés goutte à goutte 374 mL (748 mmoles) d'une solution d'iPrMgCl 2N dans le THF en maintenant la température entre -40 et -50°C. On laisse revenir le mélange réactionnel à -10°C et l'agitation est maintenue 1 h. On ajoute ensuite 172 mL (748 mmoles) de borate de triisopropyle puis on laisse revenir lentement le mélange réactionnel à T.A.. Après 2 h d'agitation, le mélange est traité avec 1 L d'une solution aqueuse d'HCl 5N puis extrait avec de l'éther (2 x 600 mL). La phase organique est lavée avec 2 x 1 L d'eau, séchée sur Na₂SO₄ puis concentrée sous pression réduite. Le résidu obtenu est concrétisé dans du pentane, filtré sur fritté et lavé avec du pentane. On obtient ainsi 113 g d'acide [3-(benzyloxy)-4-chlorophényl]boronique sous forme de solide blanc.
Rendement = 76 %
F(°C) = 148 (décomposition).

### 4.3. 6-[3-(benzyloxy)-4-chlorophényl]-5-hydroxypyridine-2-carboxylate de méthyle

Une solution de 37 g (163 mmoles) de 6-bromo-5-hydroxy-2-pyridine carboxylate de méthyle et 51 g (196 mmoles) d'acide [3-(benzyloxy)-4-chlorophényl]boronique dans 300 mL de DMF anhydre est agitée 15 min sous barbotage d'argon puis on ajoute 63,8 g (196 mmoles) de carbonate de césium anhydre et 5 g (4,33 mmoles) de Pd(PPh₃)₄. Le milieu réactionnel est agité 10 h à 90°C sous argon, refroidi à T.A. puis réparti dans 1 L d'un mélange éther/AcOEt 1:1 et 1 L d'une solution aqueuse d'HCl 0,5 N. La phase aqueuse est re-extraite avec 500 mL d'un mélange AcOEt / éther 1:1. Les phases organiques sont réunies et lavées avec 4 x 500 mL d'eau. Après séchage sur Na₂SO₄ et concentration sous pression réduite, le précipité est repris par 500 mL d'un mélange pentane / DCM 7:3, filtré et lavé avec du pentane. On obtient ainsi 32 g de 6-[3-(benzyloxy)-4-chlorophényl]-5-hydroxypyridine-2-carboxylate de méthyle sous forme de poudre jaune ocre.
Rendement = 53 %
F(°C) = 202

### 4.4. 6-[3-(benzyloxy)-4-chlorophényl]-5-{[(trifluorométhyl)sulfonyl]oxy}pyridine-2-carboxylate de méthyle

A un mélange de 38,8 g (105 mmoles) de 6-[3-(benzyloxy)-4-chlorophényl]-5-hydroxyp.yridine-2-carboxylate de méthyle dans 200 mL de DCM sont ajoutés 17,7 mL (126 mmoles) de TEA. Le mélange se solubilise progressivement et est refroidi à -5°C sous argon. On ajoute goutte à goutte 19,42 mL (115,4 mmoles) d'anhydride trifluorométhanesulfonique en maintenant la température à 0°C. Après 3 h à 0°C, le milieu réactionnel est repris avec 300 mL de DCM et lavé avec 2 x 200 mL d'eau, séché sur Na₂SO₄ puis concentrée sous pression réduite. Le résidu obtenu est purifié par chromatographie sur une colonne de silice en éluant avec un gradient pentane / AcOEt de 0 à 30 % d'AcOEt. Après concentration sous pression réduite, on obtient 47,5 g de 6-[3-(benzyloxy)-4-chlorophényl]-5-{[(trifluorométhyl)sulfonyl]oxy}pyridine-2-carboxylate de méthyle sous forme de cristaux blanc.
Rendement = 90 %
F(°C) = 89.

### 4.5. 6-[3-(benzyloxy)-4-chlorophényl]-5-(2-méthylphényl)pyridine-2-carboxylate de méthyle

Une solution de 25 g (48,8 mmoles) de 6-[3-(benzyloxy)-4-chlorophényl]-5-{[(trifluorométhyl)sulfonyl]oxy}pyridine-2-carboxylate de méthyle et de 8,8 g (64,8 mmoles) d'acide 2-méthylphénylboronique dans 200 mL de DMF anhydre est agitée 15 min sous barbotage d'argon puis on ajoute 12,7 g (60 mmoles) de phosphate de potassium (K₃PO₄) anhydre et 5,76 g (5 mmoles) de Pd(PPh₃)₄ et le mélange réactionnel est agité 18 h à 90°C sous argon. Le milieu réactionnel est ensuite réparti à T.A. dans 600 mL d'un mélange éther/AcOEt 1:1 et 600 ml d'eau. Après extraction, la phase aqueuse est re-extraite avec 100 mL d'AcOEt, les phases organiques sont réunies et lavées avec 4 x 300 mL d'eau, séchées sur Na₂SO₄ puis concentrées sous pression réduite. Le résidu obtenu est purifié par chromatographie sur une colonne de gel de silice en éluant avec un gradient heptane / AcOEt de 0 à 20 % d'AcOEt. Après concentration sous pression réduite, on obtient 18 g de 6-[3-(benzyloxy)-4-chlorophényl]-5-(2-méthylphényl)pyridine-2-carboxylate de méthyle sous forme d'huile.
Rendement = 81 %

### 4.6. 6-(4-chloro-3-hydroxy]phényl-5-(2-méthylphényl)pyridine-2-carboxylate de méthyle

A une solution de 19 g (42,8 mmoles) de 6-[3-(benzyloxy)-4-chlorophényl]-5-(2-méthylphényl)pyridine-2-carboxylate de méthyle dans 70 mL de DCM anhydre refroidie sous argon à -70°C sont ajoutés goutte à goutte en 1 h 30 85,6 mL (85,6 mmoles) d'une solution 1 N de tribromure de bore dans le DCM en maintenant la température à -65°C. Après 2 h d'agitation à -70°C, on ajoute goutte à goutte 20 mL (520 mmoles) de méthanol anhydre en maintenant la température à -65°C. Le milieu réactionnel est ramené à T.A. puis concentré sous pression réduite. Le résidu est repris par 100 mL de toluène et à nouveau concentré. L'opération est répétée encore deux fois. Le résidu obtenu est repris par 100 mL de méthanol et refroidi sous argon à 0°C puis on ajoute goutte à goutte 9 mL (128 mmoles) de chlorure de thionyle. Le milieu réactionnel est agité 48 h à T.A. puis concentré sous pression réduite. Le résidu obtenu est repris par 200 mL d'AcOEt, refroidi à 0°C et traité avec 300 mL d'une solution aqueuse saturée de NaHCO₃. Après extraction, la phase aqueuse est re-extraite avec 100 mL d'AcOEt. Les phases organiques sont réunies, lavées avec 100 mL d'eau, séchées sur Na₂SO₄ et concentrées sous pression réduite. Le résidu est purifié par chromatographie sur une colonne de gel de silice en éluant avec un gradient heptane / AcOEt de 0 à 30 % en AcOEt. Après concentration sous pression réduite, on obtient 14,7 g de 6-(4-chloro-3-hydroxyphényl)-5-(2-méthylphényl)pyridine-2-carboxylate de méthyle sous forme de poudre blanche.
Rendement = 97 %
F (°C) = 190

### 4.7. 6-{4-chloro-3-[3-(diméthylamino)propoxy]phényl}-5-(2-méthylphenyl)pyridine-2-carboxylate de méthyle

Selon le procédé décrit dans exemple 1.6, à partir de 5,4 g (15,26 mmoles) de 6-(4-chloro-3-hydroxyphényl)-5-(2-méthylphényl)pyridine-2-carboxylate de méthyle, de 9,95 g (30,53 mmoles) de carbonate de césium et de 2,9 g (18,3 mmoles) de chlorhydrate de 3-chloro-N,N-diméthylpropan-1-amine dans 60 mL de DMF, on obtient 6 g de 6-{4-chloro-3-[3-(diméthylamino)propoxy]phényl}-5-(2-méthylphényl)pyridine-2-carboxylate de méthyle sous forme d'huile.
Rendement = 90 %

### 4.8. Chlorhydrate d'acide 2-({[6-{4-chloro-3-[3-(diméthylamino)propoxylphényl}-5-(2-méthylphényl)pyridin-2-yl]carbonyl}amino)adamantane-2-carboxylique

Selon la séquence saponification/couplage peptidique décrite dans les exemples 1,7 et 1.8 respectivement, on obtient, à partir de 4,65 g (10,6 mmoles) de 6-{4-chloro-3-[3-(diméthylamino)propoxy]phényl}-5-(2-méthylphényl)pyridine-2-carboxylate de méthyle et de 2,38 g (12,18 mmoles) d'acide 2-aminoadamantane-2-carboxylique, 4,1 g de chlorhydrate d'acide 2-({[6-{4-chloro-3-[3-(diméthylamino)propoxy]phényl}-5-(2-méthylphényl)pyridin-2-yl]carbonyl}amino)adamantane-2-carboxylique sous forme de poudre blanche.
Rendement = 54 %
F(°C) : 224.
M = C₃₆H₄₀ClN₃O₄ = 601 ; M+H = 602
RMN¹H (ppm, d6-DMSO, 400MHz) : 8,50 (s, 1H) ; 8,05 (d, 1H) ; 7,90 (d, 1H) ; 7,25 (m, 5H) ; 7,10 (dd, 1H) ; 6,95 (dd, 1H) ; 3,80 (m, 2H) ; 3,1 (m, 2H) ; 2,75 (s, 6H) ; 2,60 (s, 2H) ; 1,90 (s, 3H) ; 2,2-1,6 (m, 14H).

### Exemple 5 : Chlorhydrate d'acide 2-({[6-{4-chloro-3-[3-(diméthylamino)propoxy]phényl}-5-(2-oxopyrrolidin-1-yl)pyridin-2-yl]carbonyl}amino)adamantane-2-carboxylique (composé N°23)

### 5.1. 6-[3-(benzyloxy)-4-chlorophényl]-5-(2-oxopyrrolidin-1-yl)pyridine-2-carboxylate de méthyle

Une solution de 500 mg (1 mmole) de 6-[3-(benzyloxy)-4-chlorophényl]-5-{[(trifluorométhyl)sulfonyl]oxy}pyridine-2-carboxylate de méthyle, de 0,1 mL (1,1 mmoles) de 2-pyrrolidinone et de 450 mg (1,4 moles) de carbonate de césium dans 10 mL de 1,4 dioxane anhydre est agitée 10 minutes sous barbotage d'argon puis on ajoute 9 mg (0,01 mmoles) de Pd₂(dba)₃ et 17 mg (0,03 mmoles) de Xantphos et le milieu réactionnel est chauffé 6 h à 70°C sous agitation. Le mélange est ensuite réparti dans 100 mL d'un mélange éther/AcOEt 1:1 et 50 mL d'une solution aqueuse saturée de NH₄Cl. Après extraction, la phase organique est lavée avec 2 x 50 mL d'eau, séchée sur Na₂SO₄ et concentrée sous pression réduite. Le résidu obtenu est purifié par chromatographie sur une colonne de gel de silice en éluant avec un gradient heptane / AcOEt de 0 à 30 % en AcOEt. Après concentration sous pression réduite, on obtient 293 mg de 6-[3-(benzyloxy)-4-chlorophényl]-5-(2-oxopyrrolidin-1-yl)pyridine-2-carboxylate de méthyle sous forme d'huile.
Rendement = 67 %.

### 5.2. 6-{4-chloro-3-[3-(diméthylamino)propoxy]phényl}-5-(2-oxopyrrolidin-1-yl)pyridine-2-carboxylate de méthyle

Selon la séquence débenzylation/O-alkylation décrite dans les exemples 4.6 et 1.6 respectivement, on obtient, à partir de 511 mg (1,17 mmoles) de 6-[3-(benzyloxy)-4-chlorophényl]-5-(2-oxopyrrolidin-1-yl)pyridine-2-carboxylate de méthyle, 391 mg de 6-{4-chloro-3-[3-(diméthylamino)propoxy]phényl}-5-(2-oxopyrrolidin-1-yl)pyridine-2-carboxylate de méthyle sous forme d'huile.
Rendement = 77 %

### 5.3. Chlorhydrate d'acide -2-({[6-(4-chloro-3-[3-(diméthylamino)propoxylphényl}-5-(2-oxopyrrolidin-1-yl)pyridin-2-yl]carbonyl}amino)adamantane-2-carboxylique

Selon la séquence saponification/couplage peptidique décrite dans les exemples 1.7 et 1.8 respectivement, on obtient, à partir de 391 mg (0,91 mmoles) de 6-{4-chloro-3-[3-(diméthylamino)propoxy]phényl}-5-(2-oxopyrrolidin-1-yl)pyridine-2-carboxylate de méthyle et de 265 mg (1,36 mmole) d'acide 2-aminoadamantane-2-carboxylique, 241 mg de chlorhydrate d'acide 2-({[6-{4-chloro-3-[3-(diméthylamino)propoxy]phenyl}-5-(2-oxopyrrolidin-1-yl)pyridin-2-yl]carbonyl}amino)adamantane-2-carboxylique sous forme de poudre blanche.
Rendement = 39 %
F(°C): 199
M = C₃₂H₃₈ClN₄O₅ = 594 ; M+H = 595
RMN¹H (ppm, d6-DMSO, 400 MHz) : 8,40 (s, 1H) ; 8,05 (t, 2H) ; 7,55 (d, 1H) ; 7,50 (s, 1H) ; 7,20 (dd, 1H) ; 4,15 (t, 2H) ; 3,65 (t, 2H) ; 2,75 (s, 6H) ; 2,55 (s, 2H) ; 2,2-1,6 (m, 20H).

### Exemple 6 : Chlorhydrate d'acide 2-{[(2'-{4-chloro-3-[3-(diméthylamino)propoxy]phényl}-2,3'-bipyridin-6'-yl)carbonyl]amino}adamantane-2-carboxylique (composé N°27)

### 6.1. 2'-[3-(benzyloxy)-4-chlorophényl]-2,3'-bipyridine-6'-carboxylate de méthyle

Une suspension de 0,5 g (1 mmole) de 6-[3-(benzyloxy)-4-chlorophényl]-5-{[(trifluorométhyl)sulfonyl]oxy}pyridine-2-carboxylate de méthyle, 0,44 g (1,2 mmoles) de 2-tri-n-butylstannylpyridine et 42 mg (1 mmole) de chlorure de lithium anhydre dans 2 mL de DMF anhydre est agitée 10 min sous barbotage d'argon puis on ajoute ensuite 9,5 mg (0,05 mmole) de Cul, 45 mg (0,05 mmole) de Pd₂(dba)₃ et 27 mg (0,05 mmole) de PdCl₂ (dppf) et le mélange réactionnel est alors chauffé à 90°C. Après 5 h d'agitation à 90°C, le mélange est refroidi à T.A, repris par 30 ml d'AcOEt, traité et agité pendant 15 minutes avec 30 mL d'une solution aqueuse à 5 % en poids de fluorure de potassium. Le mélange biphasique est ensuite filtré sur un gâteau de célite et la célite est rincée avec 30 mL d'AcOEt. La phase organique est lavée avec 4 x 60 mL d'eau, séchée sur Na₂SO₄ et concentrée sur pression réduite. Le résidu obtenu est purifié par chromatographie sur une colonne de silice en éluant avec un gradient toluène / AcOEt de 0 à 15 % en AcOEt. Après concentration sous pression réduite, on obtient 240 mg de 2'-[3-(benzyloxy)-4-chlorophényl]-2,3'-bipyridine-6'-carboxylate de méthyle sous forme d'huile.
Rendement = 55 %

### 6.2. 2'-{4-chloro-3-[3-(diméthylamino)propoxy]phényl}-2,3'-bipyridine-6'-carboxylate de méthyle

Selon la séquence débenzylation/O-alkylation décrite dans les exemples 4.6 et 1.6 respectivement, on obtient, à partir de 680 mg (1,58 mmoles) de 2'-[3-(benzyloxy)-4-chlorophényl]-2,3'-bipyridine-6'-carboxylate de méthyle, 580 mg de 2'-{4-chloro-3-[3-(diméthylamino)propoxy]phényl}-2,3'-bipyridine-6'-carboxylate de méthyle sous forme d'huile.
Rendement = 86 %

### 6.3. Chlorhydrate d'acide 2-{[(2'-{4-chloro-3-[3-(diméthylamino)-propoxy]phényl}-2,3'-bipyridin-6'-yl)carbonyl]amino}adamantane-2-carboxylique

Selon la séquence saponification/couplage peptidique décrite dans les exemples 1.7 et 1.8 respectivement, on obtient, à partir de 581 mg (1,36 mmoles) de 2'-{4-chloro-3-[3-(diméthylamino)propoxy]phényl}-2,3'-bipyridine-6'-carboxylate de méthyle et de 365 mg (1,87 mmole) d'acide 2-aminoadamantane-2-carboxylique, 435 mg de chlorhydrate d'acide 2-{[(2'-{4-chloro-3-[3-(diméhylamino)propoxy]phényl}-2,3'-bipyridin-6'-yl)carbonyl]amino}adamantane-2-carboxylique sous forme de poudre blanche.
Rendement = 48 %
F(°C) : 209
M = C₃₃H₃₇ClN₄O₄ = 588 ; M+H = 589
RMN¹H (ppm, d6-DMSO, 400 MHz) : 10,55 (s, 1H) ; 8,65 (s, 1H) ; 8,50 (s, 1H) ; 8,25 (d, 1H) ; 8,10 (d, 1 H) ; 7,80 (t, 1 H) ; 7,40 (dd, 1 H) ; 7,35 (d, 2H) ; 7,25 (s, 1H) ; 6,80 (d, 1H) ; 3,95 (t, 2H) ; 3,15 (m, 2H) ; 2,75 (s, 3H) ; 2,70 (s, 3H) ; 2,60 (s, 2H) ; 2,2-1,8 (m, 14H).

### Exemple 7 : Chlorhydrate d'acide 2-({[6-{4-chloro-3-[3-(diméthylamino)propoxy)phényl}-5-(2-chlorophényl)pyridin-2-yl]carbonyl}amino)adamantane-2-carboxylique (composé N° 22)

### 7.1. 6-{4-chloro-3-[3-(diméthylamino)propoxy]phényl}-5-(2-chlorophényl)pyridine-2-carboxylate de méthyle

Selon la séquence débenzylation/O-alkylation décrite dans les exemples 4.6 et 1.6 respectivement, on obtient, à partir de 625 mg (1,35 mmoles) de 6-[3-(benzyloxy)-4-chlorophényl]-5-(2-chlorophényl)pyridine-2-carboxylate de méthyle, 173 mg de 6-{4-chloro-3-[3-(diméthylamino)propoxy]phényl}-5-(2-chlorophényl)pyridine-2-carboxylate de méthyle sous forme d'huile.
Rendement = 28 %

### 7.2. Chlorhydrate d'acide 2-({[6-{4-chloro-3-[3-(diméthylamino)propoxy]phényl}-5-2-chlorophényl)pyridin-2-yl]carbonyl}amino)adamantane-2-carboxylique

Selon la séquence saponification/couplage peptidique décrite dans les exemples 1.7 et 1.8 respectivement, on obtient, à partir de 290 mg (0,63 mmoles) de 6-{4-chloro-3-[3-(diméthylamino)propoxy]phényl}-5-(2-chlorophényl)pyridine-2-carboxylate de méthyle et de 184 mg (0,94 mmole) d'acide 2-aminoadamantane-2-carboxylique, 120 mg de chlorhydrate d'acide 2-({[6-{4-chloro-3-[3-(diméthylamino)propoxy]phényl}-5-(2-chlorophényl)pyridin-2-yl]carbonyl}aimino)adamantane-2-carboxylique sous forme de poudre blanche.
Rendement = 29 %
F(°C) : 214
M = C₃₄H₃₇Cl₂N₃O₄ = 621 ; M+H = 622
RMN¹H (ppm, d6-DMSO, 400MHz) : 8,50 (s, 1 H) ; 8,10 (d, 1H) ; 8,00 (d, 1H) ; 7,45 (m, 2H) ; 7,40 (d, 2H) ; 7,35 (d, 1H) ; 7,20 (dd, 1H) ; 6,90 (dd, 1H) ; 3,90 (m, 2H) ; 3,15 (m, 2H) ; 2,75 (s, 6H) ; 2,60 (s, 2H) ; 2,2-1,6 (m, 14H).

### Exemple 8 :Chlorhydrate d'acide 1-({[6-{4-chloro-3-[3-(diméthylamino)propoxy]phényl}-5-(2-méthylphényl)pyridin-2-yl]carbonyl}amino)cyclohexanecarboxylique (composé N°34)

Selon la séquence saponifcation/couplage peptidique décrite dans les exemples 1.7 et 1.8 respectivement, on obtient, à partir de 465 mg (1,06 mmoles) de 6-{4-chloro-3-[3-(diméthylamino)propoxy]phényl}-5-(2-méthylphényl)pyridine-2-carboxylate de méthyle (exemple 4.7) et de 174 mg (1,22 mmoles) d'acide 2-aminocyclohexane-2-carboxylique, 330 mg de chlorhydrate de chlorhydrate d'acide 1-({[6-{4-chloro-3-[3-(diméthylamino)propoxy]phényl}-5-(2-méthylphényl)pyridin-2-yl]carbonyl}amino)cyclohexanecarboxylique sous forme de poudre blanche.
Rendement = 53 %
F (°C) = 146-150
M = C₃₁H₃₆ClN₃O₄ = 549 ; M+H = 550
RMN¹H (ppm, d6-DMSO, 400 MHz) : 10,5 (s, 1H) ; 8,40 (s, 1H) ; 8,05 (d, 1H) ; 7,90 (d, 1H) ; 7,35 (d, 1 H) ; 7,25 (m, 5H) ; 7,05 (dd, 1 H) ; 3,80 (m, 2H) ; 3,1 (m, 2H) ; 2,75 (s, 6H) ; 1.75 (s, 3H) ; 2,2-1,6 (m, 12H).

### Exemple 9 : Chlorhydrate d'acide cis-1-({[6-{4-chloro-3-[3-(diéthylamino)propoxy]phényl}-5-(2-méthylphenyl)pyridin-2-yl]carbonyl}amino)-4-hydroxycyclohexane carboxylique (composé N°147)

A un mélange de 490 mg (1 mmole) de chlorhydrate d'acide 6-{4-chloro-3-[3-(diéthylamino)propoxy]phényl}-5-(2-méthylphényl)pyridine-2-carboxylique dans 5 mL de DMF anhydre, on ajoute successivement sous argon et à T.A., 127 mg (1,1 mmoles) de N-hydroxysuccinimide et 211 mg (1,1 mmoles) d'EDC.HCl. Après 18 h d'agitation on ajoute successivement au milieu réactionnel (jaune vif et limpide) 0,9 mL (5,4 mmoles) de DIEA et 215 mg (1,1 mmoles) d'acide cis-1-amino-4-hydroxycyclohexane carboxylique (J.Chem.Soc., Perkin Trans. 1 (1999) pp. 3375-3379). L'agitation est poursuivie 18 h à T.A. puis le mélange réactionnel est concentré sous pression réduite. Le résidu obtenu est ensuite traité 18 h à T.A. avec 7 mL (7 mmoles) d'HCl 1 N puis concentré sous pression réduite. Le résidu est purifié par HPLC sur une colonne de RP18 en éluant avec un gradient HCl 10⁻²N / acétonitrile de 0% à 100% d'acétonitrile. Après lyophilisation, on obtient 350 mg de chlorhydrate d'acide *cis*-1-({[6-{4-chloro-3-[3-(diéthylamino)propoxy]phényl}-5-(2-méthylphényl)pyridin-2-yl]carbonyl}amino)-4-hydroxycyclohexane carboxylique sous forme de poudre blanche.
Rendement = 55 %
F (°C) = 168
M = C₃₃H₄₀ClN₃O₅ = 565 ; M+H = 566
RMN¹H (ppm, d6-DMSO, 400 MHz) : 8,55 (s, 1H) ; 8,05 (d, 1H) ; 7,90 (d, 1H) ; 7,25 (m, 6H) ; 6,90 (dd, 1 H) ; 4,65 (sl, 1 H) ; 3,90 (m, 2H) ; 3,50 (m, 1H) ; 3,10 (m, 2H) ; 2,95 (q, 4H) ; 2,30 (m, 2H) ; 2,05 (m, 2H) ; 1,90 (s, 3H) ; 1,80 (m, 4H) ; 1,35 (m, 2H) ; 1,10 (t, 6H)

### Exemple 10 : Chlorhydrate d'acide 3-[({4"-chloro-3"-[3-(diméthylamino)propoxy]-2,6-diméthoxy-1,1':2',1"-terphényl-4'-yl}carbonyl)amino]-4-méthylpentanoique (composé N°28)

### 10.1. 2-[3-(benzyloxy)-4-chlorophényl]-4,4,5,5-tétraméthyl-1,3,2-dioxaborolane

Une suspension de 20 g (67,2 mmoles) de 2-benzyloxy-4-bromo-1-chlorobenzène, de 28 g (94 mmoles) de 4,4,4',4',5,5,5',5'-octaméthyl-2,2'-bi-1,3,2-dioxaborolane et de 26,4 g (26,9 mmoles) d'acétate de potassium dans 280 mL de DMSO anhydre est agitée 10 minutes sous argon, puis on ajoute 2,46 g (3,4 mmoles) de PdCl₂ (dppf) et on chauffe 1 h. à 110°C. Après répartition dans 1 L d'un mélange éther/eau 1:1 suivie d'une filtration sur gâteau de célite, la phase organique est lavée avec 100 mL d'eau, séchée sur Na₂SO₄ puis concentrée sous pression réduite. Le résidu obtenu est purifié par chromatographie sur une colonne de gel de silice en éluant avec un gradient heptane/AcOEt de 0 à 10 % d'AcOEt. Après concentration sous pression réduite, on obtient 17, 5 g de 2-[3-(benzyloxy)-4-chlorophényl]-4,4,5,5-tétraméthyl-1,3,2-dioxaborolane sous forme d'huile.
Rendement = 74 %.

### 10.2. 2-bromo-2',6'-diméthoxybiphényl-4-carboxylate de méthyle

Une solution de 4,84 g (14,2 mmoles) de 3-bromo-4-iodobenzoate de méthyle (J. Med. Chem., 1999, 42, 4088) et de 3,88 g (21,29 mmoles) d'acide 2,6-diméthoxyphényl boronique dans 120 mL de DMF et 14,2 mL d'une solution aqueuse 2 M de carbonate de césium est agitée 15 minutes sous argon, puis on ajoute 984 mg (0,85 mmole) de Pd(PPh₃)₄ et on chauffe 2,5 h. à 85°C. Après concentration sous pression réduite, le résidu obtenu est réparti dans 600 mL d'un mélange DCM/eau 1:1. La phase organique est lavée avec 100 mL. d'eau, séchée sur MgSO₄ et concentrée sur pression réduite. Le résidu obtenu est purifié par chromatographie sur une colonne de gel de silice en éluant avec un gradient heptane / AcOEt de 0 à 10 % en AcOEt. Après concentration sous pression réduite, on obtient 2,79 g de méthyl 2-bromo-2',6'-diméthoxybiphényl-4-carboxylate sous forme d'huile.
Rendement = 56 %

### 10.3. 3"-(benzyloxy)-4"-chloro-2,6-diméthoxy-1,1':2',1"-terphényl-4'-carboxylate de méthyle

Selon le procédé décrit dans l'exemple 10.2, on obtient à partir de 2,79 g (7,94 mmoles) de 2-bromo-2',6'-diméthoxybiphényl-4-carboxylate de méthyle et de 3,28 g (9,53 mmoles) de 2-[3-(benzyloxy)-4-chlorophényl]-4,4,5,5-tétraméthyl-1,3,2-dioxaborolane, 1,75 g de 3"-(benzyloxy)-4"-chloro-2,6-diméthoxy-1,1':2',1"-terphényl-4'-carboxylate de méthyle sous forme d'huile.
Rendement = 45 %

### 10.4. 4"-chloro-3"-[3-(diméthylamino)propoxy]-2,6-diméthoxy-1,1':2',1"-terphenyl-4'-carboxylate de méthyle

Selon la séquence débenzylation/O-alkylation décrite dans les exemples 4.6 et 1.6 respectivement, on obtient, à partir de 1,75 g (3,58 mmoles) de 3"-(benzyloxy)-4"-chloro-2,6-diméthoxy-1,1':2',1"-terphényl-4'-carboxylate de méthyle, 900 mg de 4"-chloro-3"-[3-(diméthylamino)propoxy]-2,6-diméthoxy-1,1':2',1"-terphenyl-4'-carboxylate de méthyle sous forme d'huile.
Rendement = 52 %

### 10.5. Acide 4"-chloro-3"-[3-(diméthylamino)propoxyl]-2,6-diméthoxy-1,1':2',1"-terphényl-4'-carboxylique.

Selon le procédé décrit dans l'exemple 1.7, on obtient, à partir de 900 mg (1,88 mmoles) de 4"-chloro-3"-[3-(diméthylamino)propoxy]-2,6-diméthoxy-1,1':2',1"-terphenyl-4'-carboxylate de méthyle, 700 mg d'acide 4"-chloro-3"-[3-(diméthylamino)propoxyl-2,6-diméthoxy-1,1':2',1"-terphényl-4'-carboxylique sous forme de solide rosé.
Rendement = 82 %
F(°C) = 230.

### 10.6. Chlorhydrate d'acide 3-[({4"-chloro-3"-[3-diméthylamino)propoxy]-2,6-diméthoxy-1,1':2',1"-terphényl-4'-yl}carbonyl)amino]-4-méthylpentanoique

A une solution de 315 mg (0,67 mmole) d'acide 4"-chloro-3"-[3-(diméthylamino)propoxy]-2,6-diméthoxy-1,1':2',1"-terphényl-4'-carboxylique dans 7 mL de THF anhydre, on ajoute 141 mg (0,87 mmole) de CDl et le milieu réactionnel est agité à 55°C pendant 1 h. sous argon. On ajoute 70 mg (0,43 mmole) de carbonyl-1,1'-diimidazole et la réaction est poursuivie 1 h à 50°C. On ajoute alors une suspension de 97 mg (0,74 mmole) d'acide 3-amino-4-méthylpentanoique racémique dans un mélange de 4 mL de THF et 0,8 mL de DMF et l'agitation est poursuivie 15 h. à 55°C. Après concentration sous pression réduite, le résidu obtenu est réparti dans 30 mL d'un mélange DCM/eau 2:1. La phase organique est lavée avec 10 mL d'eau, séchée sur sulfate de magnésium (MgSO₄) et concentrée sous pression réduite. Le résidu obtenu est purifié par chromatographie sur une colonne de gel de silice en éluant avec un gradient DCM / méthanol de 0 à 5 % en méthanol. Après concentration sous pression réduite, le résidu est concrétisé dans de l'éther, filtré sur fritté et lavé avec de l'éther. On obtient ainsi 91 mg de chlorhydrate d'acide 3-[({4"-chloro-3"-[3-(diméthylamino)propoxy]-2,6-diméthoxy-1,1':2',1"-terphényl-4'-yl}carbonyl)amino]-4-méthylpentanoique sous forme de poudre blanche.
Rendement = 23 %
F(°C) = 152
M = C₃₂H₃₉ClN₂O₆ = 582 ; M+H = 583
RMN ¹H (ppm, d6-DMSO, 400 MHz) : 8,3 (d, 1H) ; 7,75 (d, 1H) ; 7,7 (s, 1H) ; 7,2 (m, 3H) ; 6,7 (m, 2H) ; 6,55 (d, 2H) ; 4,1 (q, 1H) 3,8 (t, 2H) ; 3,5 (s, 6H) ; 2,45 (m, 4H) ; 2,3 (s, 6H) 1,8 (m, 3H) ; 0,9 (d, 6H).

### Exemple 11 : Chlorhydrate d'acide 3-({[5-{4-chloro-3-[3-(diméthylamino)propoxy]phényl}-6-(2-méthylphényl)pyridin-3-yl]carbonyl}amino)-4-méthylpentanoique (composé 29)

### 11.1. 5-[3-(benzyloxy)-4-chlorophényl]-6-oxo-1,6-dihydropyridine-3-carboxylate de méthyle

Une solution de 4 g (14,35 mmoles) de 5-iodo-6-oxo-1,6-dihydropyridine-3-carboxylate de méthyle et de 6,42 g (18,64 mmoles) de 2-[3-(benzyloxy)-4-chlorophényl]-4,4,5,5-tétraméthyl-1,3,2-dioxaborolane dans un mélange de 160 mL de diméthoxyéthane (DME), 80 mL d'EtOH et de 120 mL d'une solution aqueuse saturée en NaHCO₃ est agitée 15 minutes sous argon, puis on ajoute 662 mg de Pd(PPh₃)₄ et le mélange réactionnel est chauffé 4 h30 à 90°C. Après concentration sous pression réduite, le résidu obtenu est réparti dans un mélange de 200 mL de DCM et 10 mL d'eau. La phase aqueuse est re-extraite avec 100 mL de DCM, les phases organiques sont réunies, séchées sur MgSO₄, filtrées et concentrées sous pression réduite. Le résidu obtenu est purifié par chromatographie sur une colonne de gel de silice en éluant avec un gradient DCM / méthanol de 0 à 2 % en méthanol. Après concentration sous pression réduite, le résidu est concrétisé dans un mélange méthanol / éther 5/95 puis filtré et lavé avec de l'éther. On obtient ainsi 1,99 g de 5-[3-(benzyloxy)-4-chlorophényl]-6-oxo-1,6-dihydropyridine-3-carboxylate de méthyle sous forme de solide beige.
Rendement = 40 %
F(°C) : 190

### 11.2. 5-[3-(benzyloxy)-4-chlorophényl]-6-{[(trifluorométhyl)sulfonyl]oxy}nicotinate de méthyle

Selon le procédé décrit dans l'exemple 4.4, on obtient à partir de 1,99 g (5,38 mmoles) de 5-[3-(benzyloxy)-4-chlorophényl]-6-oxo,1,6-dihydropyridine-3-carboxylate de méthyle et 2,26 ml (13,45 mmoles) d'anhydre trifluorométhanesulfonique, 1,6 g de 5-[3-(benzyloxy)-4-chlorophényl]-6-([(trifluorométhyl)sulfonyl]oxy}nicotinate de méthyle sous forme d'huile.
Rendement = 60 %

### 11.3. 5-[3-(benzyloxy)-4-chlorophényl]-6-(2-méthylphényl)nicotinate de méthyle

Selon le procédé décrit dans l'exemple 4.5, on obtient à partir de 1,5 g (2,99 mmoles) de 5-[3-(benzyloxy)-4-chlorophényl]-6-{[(trifluorométhyl)sulfonyl]oxy}nicotinate de méthyle et de 508 mg (3,74 mmoles) d'acide 2-méthylphényl boronique, 1,26 g de 5-[3-(benzyloxy)-4-chlorophényl]-6-(2-méthylphényl)nicotinate de méthyle sous forme d'huile.
Rendement = 95 %

### 11.4. 5-{4-chloro-3-[3-(diméthylamino)propoxy]phényl}-6-(2-méthylphényl)nicotinate de méthyle

Selon la séquence débenzylation/O-alkylation décrite dans les exemples 4.6 et 1.6 respectivement, on obtient, à partir de 1,26 g (2,84 mmoles) de 5-[3-(benzyloxy)-4-chlorophényl]-6-(2-méthylphényl)nicotinate de méthyle, 843 mg de 5-{4-chloro-3-[3-(diméthylamino)propoxy]phényl}-6-(2-méthylphényl)nicotinate de méthyle sous forme d'huile.
Rendement = 68 %

### 11.5. Chlorhydrate d'acide 3-({[5-{4-chloro-3-[3-(diméthylamino)propoxyl]phényl}-6-(2-méthylphényl)pyridin-3-yl]carbonyl}amino)-4-méthylpentanoique

Selon la séquence saponification/couplage peptidique décrite dans les exemples 1.7 et 10.6 respectivement, on obtient à partir de 835 mg (1,90 mmoles) de 5-{4-chloro-3-[3-(diméthylamino)propoxy]phényl}-6-(2-méthylphényl)nicotinate de méthyle et de 258 mg (1,97 mmoles) d'acide 3-amino-4-méthylpentanoïque racémique, 130 mg de chlorhydrate d'acide 3-({[5-{4-chloro-3-[3-(diméthylamino)propoxy]phényl}-6-(2-méthylphényl)pyridin-3-yl]carbonyl}amino)-4-méthylpentanoique sous forme de solide blanc.
Rendement = 13 %
F(°C) = 139
M = C₃₀H₃₆ClN₃O₄ = 537 ; M+H = 538
RMN¹H (ppm, d6-DMSO, 400 MHz) : 9,05 (d, 1H) ; 9,5 (d, 1H) ; 9,25 (d,1H) ; 8,3 (d, 1H) ; 7,3 (d, 1 H) 7.15 (d, 1H) ; 7,15 (m, 4H) ; 4,25 (m, 1H) ; 3,8 (t, 2H) ; 2,65 (t, 2H) ; 2,45 (s, 6H) 1,95 (s, 3H) ; 1,85 (m, 5H) ; 0,90 (d, 6H).

### Exemple 12 : Chlorhydrate d'acide 2-({[6-[3-[3-(diméthylamino)propoxy]-4-(trifluorométhyl)phényl]-5-(2-méthylphényl)pyridin-2-yl]carbonyl}amino)adamantane-2-carboxylique (composé N°43)

### 12.1. 2-iodo-5-nitrophénol

A une solution de 30,81 g (200 mmoles) de 2-amino-5-nitrophénol dans un mélange de 500 mL d'acide sulfurique à 30 % en poids et 500 mL de DMSO refroidie à 5°C est ajoutée goutte à goutte une solution de 20,7 g (300 mmoles) de nitrite de sodium (NaNO₂) dans 100 mL d'eau en maintenant la température à 5°C. Après une demi-heure à 5°C, on ajoute goutte à goutte une solution de 90 g (600 mmoles) d'iodure de sodium dans 100 mL d'eau puis le milieu réactionnel est ramené à T.A.. Après 2 h d'agitation, le milieu réactionnel est réparti dans 2 L d'un mélange éther/solution aqueuse à 10 % en poids d'hydrogénosulfite de sodium 1:1. La phase aqueuse est re-extraite avec 200 mL d'éther et les phases organiques réunies sont lavées avec 3 x 1 L d'eau puis séchées sur Na₂SO₄. Après concentration sous pression réduite, le résidu obtenu est purifié par chromatographie sur une colonne de gel de silice en éluant avec un gradient cyclohexane / AcOEt de 0 à 10 % en AcOEt. Après concentration sous pression réduite, on obtient 39,8 g de 2-iodo-5-nitrophénol sous forme de solide marron.
Rendement = 75 %
F(°C) = 150

### 12.2. 2-(benzyloxy)-1-iodo-4-nitrobenzène

Selon le procédé décrit dans l'exemple 4.1., on obtient, à partir de 20,8 g (78,5 mmoles) de 2-iodo-5-nitrophénol et 9,3 mL (78,5 mmoles) de bromure de benzyle, 25,2 g de 2-(benzyloxy)-1-iodo-4-nitrobenzène sous forme d'huile.
Rendement = 90 %

### 12.3. 2-(benzyloxy)-4-nitro-1-(trifluorométhyl)benzéne

A un mélange de 4,08 g (70,2 mmoles) de fluorure de potassium anhydre et de 13,36 g (70,18 mmoles) de Cul anhydre et 19,17 g (54 mmoles) de 2-(benzoxy)-1-iodo-nitrobenzène dans 77 mL de NMP anhydre, on ajoute 10,4 mL (70,18 mmoles) de trifluorométhyltriméthylsilane et le milieu réactionnel est chauffé 18 h à 45°C sous argon. La suspension est répartie à T.A. dans 1 L d'un mélange éther/eau : 1:1, la phase organique est ensuite lavée avec 4 x 300 mL d'eau, séchée sur Na₂SO₄, puis concentrée sous pression réduite. On obtient ainsi 15 g de 2-(benzyloxy)-4-nitro-1-(trifluorométhyl)benzène sous forme d'huile.
Rendement = 94 %

### 12.4. 3-(benzyloxy)-4-(trifluorométhyl)aniline

A un mélange de 15 g (50,3 mmoles) de 2-(benzyloxy)-4-nitro-1-(trifluorométhyl)benzène dans 200 mL d'EtOH, 10 mL d'AcOH et 100 mL d'eau sont ajoutés 14,1 g (252 mmoles) de limaille de fer puis le mélange réactionnel est placé sous forte agitation à 70°C pendant 20 minutes. Après refroidissement à T.A., le mélange réactionnel est additionné lentement sur un mélange de 300 mL d'éther et 1 L d'une solution aqueuse saturée en Na₂CO₃ Après neutralisation, la phase aqueuse est re-extraite avec 2x100 mL d'éther. Les phases organiques sont réunies, lavées avec 100 mL d'eau, séchées sur chlorure de calcium et concentrées sous pression réduite. Le résidu obtenu est purifié par chromatographie sur une colonne de gel de silice en éluant avec un gradient pentane / AcOEt de 0 à 50 % d'AcOEt. Après concentration sous pression réduite, on obtient 8,1 g de 3-(benzyloxy)-4-(trifluorométhyl)aniline sous forme d'huile.
Rendement = 60 %

### 12.5. 2-(benzyloxy)-4-iodo-1-(trifluorométhyl)benzène

Selon le procédé décrit dans l'exemple 12.1., on obtient, à partir de 8 g (30 mmoles) de 3-(benzyloxy)-4-(trifluorométhyl)aniline, 8 g de 2-(benzyloxy)-4-iodo-1-(trifluorométhyl)benzène sous forme d'huile.
Rendement = 70%

### 12.6. Acide [3-(benzyloxy-4-(trifluorométhyl)phényl]boronique

Selon le procédé décrit dans l'exemple 4.2, on obtient, à partir de 8 g (21,2 mmoles) de 2-(benzyloxy)-4-iodo-1-(trifluorométhyl)benzène, 11,1 mL (22,2 mmoles) d'iPrMgCl et 5 mL (21,6 mmoles) de borate de triisopropyle, 4,95 g d'acide [3-(benzyloxy)-4-(trifluorométhyl)phényl]boronique sous forme d'huile.
Rendement = 79 %

### 12.7. 6-[3-(benzyloxy)-4-(trifluorométhyl)phényl]-5-hydroxypyridine-2-carboxylate de méthyle

Selon le procédé décrit dans l'exemple 4.3, on obtient, à partir de 4,95 g (16,7 mmoles) d'acide [3-(benzyloxy)-4-(trifluorométhyl)phényl]boronique et 3,88 g (16,72 mmoles) de 6-bromo-5-hydroxypyridine-2-carboxylate de méthyle, 4,38 g de 6-[3-(benzyloxy)-4-(trifluorométhyl)phényl]-5-hydroxypyridine-2-carboxylate de méthyle sous forme de solide blanc.
Rendement = 65 %
F(°C) = 82

### 12.8. 6-[3-(benzyloxy)-4-(trifluorométhyl)phényl]-5-(2-méthylphényl)pyridine-2-carboxylate de méthyle

Selon la séquence triflate/couplage de Suzuki décrite dans les exemples 4.4 et 4.5 respectivement, on obtient, à partir de 4,38 g (10,9 mmoles) de 6-[3-(benzyloxy)-4-(trifluorométhyl)phényl]-5-hydroxypyridine-2-carboxylate de méthyle et 1,8 g (13,1 mmoles) d'acide 2-méthylphénylboronique, 4,63 g de 6-[3-(benzyloxy)-4-(trifluorométhyl)phényl]-5-(2-méthylphényl)pyridine-2-carboxylate de méthyle sous forme de solide blanc
Rendement = 89 %
F(°C) = 226

### 12.9. 6-[3-[3-(diméthylamino)propoxy]-4-(trifluorométhyl)phényl]-5-(2-méthylphényl)pyridine-2-carboxylate de méthyle

Selon la séquence débenzylation/O-alkylation décrite dans les exemples 4.6 et 1.6 respectivement, on obtient à partir de 2,57 g (5,38 mmoles) de 6-[3-(benzyloxy)-4-(trifluorométhyl)phényl]-5-(2-méthylphényl)pyridine-2-carboxylate de méthyle, 2,26 g de 6-[3-[3-(diméthylamino)propoxy]-4-(tnfluorométhyl)phényl]-5-(2-méthylphényl)pyridine-2-carboxylate de méthyle sous forme de solide blanc.
Rendement = 89 %
F(°C) = 142

### 12.10. Chlorhydrate d'acide 2-({[6-[3-[3-(diméthylamino)propoxy]-4-(trifluorométhyl)phényl]-5-(2-méthylphényl)pyridin-2-yl]carbonyl}amino)adamantane-2-carboxylique

Selon la séquence saponification/couplage peptidique décrite dans les exemples 1.7 et 1.8 respectivement, on obtient, à partir de 565 mg (1,19 mmoles) de 6-[3-[3-(diméthylamino)propoxy]-4-(trifluorométhyl)phényl]-5-(2-méthylphényl)pylidine-2-carboxylate de méthyle et 256 mg (1.31 mmoles) d'acide 2-amino-adamantane-2-carboxylique, 440 mg de chlorhydrate d'acide 2-({[6-[3-[3-(diméthylamino)propoxy]-4-(trifluorométhyl)phényl]-5-(2-méthylphényl)pyridin-2-yl]carbonyl}amino)adamantane-2-carboxylique sous forme de poudre blanche.
Rendement = 55 %
F(°C) = 231
M = C₃₈H₄₀F₃N₃O₄ = 634 ; M+H = 635
RMN¹H (ppm, d6-DMSO, 400 MHz) : 8,50 (s, 1 H) ; 8,10 (d, 1 H) ; 7,95 (d, 1H) ; 7,55 (d, 1H) : 7,25 (m, 5H) ; 7,10 (d, 1H); 3,90 (m, 2H) ; 3,05 (m, 2H) ; 2,70 (s, 6H) ; 2,60 (s, 2H) ; 2,05 (m, 7H) ; 1,80 (s, 3H) ; 1,65 (m, 7H).

### Exemple 13 : Chlorhydrate d'acide 1-{[(3-chloro-2'-{4-chloro-3-[3-(diméthylamino)propoxy]phényl}-2,3'-bipyridin-6'-yl)carbonyl]amino}cyclohexane carboxylique (composé n°55)

### 13.1. 6-[3-(benzyloxy)-4-chlorophényl]-5-(4,4,5,5-tétraméthyl-1,3,2-dioxaborolan-2-yl)pyridine-2-carboxylate de méthyle

Une solution de 5 g (10 mmoles) de 6-[3-(benzyloxy)-4-chlorophényl]-5-{[(trifluorométhyl)sulfonyl]oxy}pyridine-2-carboxylate de méthyle et de 2,78 g (11 mmoles) de 4,4,4',4',5,5,5',5'-octaméthyl-2,2'-bi-1,3,2-dioxaborolane dans 50 mL de 1,4-dioxane anhydre est agitée 15 min sous barbotage d'argon puis on ajoute 2,93 g (30 mmoles) d'acétate de potassium anhydre et 0,37 g (0,45 mmoles) de PdCl₂(dppf et on chauffe le mélange réactionnel 26 h à 80°. Le mélange réactionnel est ensuite réparti dans 100 mL d'un mélange AcOEt/saumure 1:1. La phase organique est séchée sur Na₂SO₄, filtrée puis concentrée sous pression réduite. Le résidu obtenu est purifié par chromatographie sur une colonne de silice en éluant avec un gradient cyclohexane / AcOEt de 0 à 20 % d'AcOEt. Après concentration sous pression réduite, on obtient 3,2 g de 6-[3-(benzyloxy)-4-chlorophényl]-5-(4,4,5,5-tétraméthyl-1,3,2-dioxaborolan-2-yl)pyridine-2-carboxylate de méthyle sous forme d'une cire.
Rendement = 67 %

### 13.2. 2'-[3-(benzyloxy)-4-chlorophényl]-3-chloro-2,3'-bipyridine-6'-carboxylate de méthyle

Une solution de 850 mg (1,77 mmoles) de 6-[3-(benzyloxy)-4-chlorophényl]-5-(4,4,5,5-tétraméthyl-1,3,2-dioxaborolan-2-yl)pyridine-2-carboxylate de méthyle et de 524 mg (3,54 mmoles) de 2,3-dichloropyridine dans un mélange de 4 mL de DME et 2 mL d'eau est agitée 15 minutes sous argon puis on ajoute successivement 734 mg (5,32 mmoles) de K₂CO₃ et 61 mg (0,05 mmoles) de Pd(PPh₃)₄ et on chauffe le milieu réactionnel 4 h. à 85°C. Le mélange réactionnel est ensuite réparti entre 10 mL d'AcOEt et 10 mL de saumure. La phase organique est séchée sur Na₂SO₄, filtrée puis concentrée sous pression réduite. Le résidu obtenu est purifié sur une colonne de silice en éluant avec un gradient cyclohexane/AcOEt de 0 à 20% d'AcOEt. Après concentration sous pression réduite, on obtient 237 mg de 2'-[3-(benzyloxy)-4-chlorophényl]-3-chloro-2,3'-bipyridine-6'-carboxylate de méthyle sous forme d'huile.
Rendement = 28 %

### 13.3. 3-chloro-2'-{4-chloro-3-[3-(diméthylamino)propoxy]phényl}-2,3'-bipyridine-6'-carboxylate de méthyle

Selon la séquence débenzylation/O-alkylation décrite dans les exemples 4.6 et 1.6 respectivement on obtient, à partir de 360 mg (0,77 mmoles) de 2'-[3-(benzyloxy)-4-chlorophényl]-3-chloro-2,3'-bipyridine-6'-carboxylate de méthyle, 130 mg de 3-chloro-2'-{4-chloro-3-[3-(diméthylamino)propoxy]phényl}-2,3'-bipyridine-6'-carboxylate de méthyle sous forme d'huile.
Rendement = 37 %

### 13.4. Chlorhydrate d'acide 1-{[(3-chloro-2'-{4-chloro-3-[3-(diméthylamino)propoxy]phényl}-2,3'-bipyridin-6'-yl)carbonyl]amino}cyclohexane carboxylique

Selon la séquence saponification/couplage peptidique décrite dans les exemples 1.7 et 1.8 respectivement, on obtient, à partir de 130 mg (0,28 mmoles) de 3-chloro-2'-{4-chloro-3-[3-(diméthylamino)propoxy]phényl}-2,3'-bipyridine-6'-carboxylate de méthyle et 44 mg (0,31 mmoles) d'acide 2-aminocyclohexanecarboxylique, 120 mg de chlorhydrate d'acide 1-{[(3-chloro-2'-{4-chloro-3-[3-(diméthylamino)propoxy]phényl}-2,3'-bipyridin-6'-yl)carbonyl]amino}cyclohexane carboxylique sous forme de poudre blanche.
Rendement = 70 %
F(°C) = 140
M = C₂₉H₃₂Cl₂N₄O₄ = 570 ; M+H = 571
RMN¹H (ppm, d6-DMSO, 400 MHz) : 10,45 (s, 1H) ; 8,65 (d, 1H) ; 8,45 (s, 1H) ; 8,10 (s, 2H) ; 7,95 (d, 1 H) ; 7,50 (dd, 1 H) ; 7,35 (d, 1 H) ; 7,25 (d, 1H) ; 6,90 (dd, 1H) ; 3,95 (t, 2H) ; 3,15 (m, 2H) ; 2,80 (d, 6H) ; 2,20 (m, 4H) ; 1,80 (t, 2H) ; 1,70-1,2 (m, 6H).

### Exemple 14 : Chlorhydrate d'acide (3S)-3-({[6-(4-chloro-3-[3-(diméthylamino)propoxy]phényl}-5-(2-chloro-5-méthylphényl)pyridin-2-yl]carbonyl}amino)-4-méthylpentanoique (composé N°72)

### 14.1. 6-{4-chloro-3-[3-(diméthylamino)propoxy]phényl}-5-(2-chloro-5-méthylphényl)pyridine-2-carboxylate de méthyle

Selon la séquence débenzylation/O-alkylation décrite dans les exemples 4.6 et 1.6 respectivement on obtient, à partir de 743 mg (1,55 mmoles) de 6-[3-(benzyloxy)-4-chlorophényl]-5-(2-chloro-5-méthylphényl)pyridine-2-carboxylate de méthyle, 530 mg de 6-{4-chloro-3-[3-(diméthylamino)propoxy]phényl}-5-(2-chloro-5-méthylphényl)pyridine-2-carboxylate de méthyle sous forme d'huile.
Rendement = 72%

### 14.2. Chlorhydrate d'acide (3S)-3-({[6-{4-chloro-3-[3-(diméthylamino)propoxy]phényl}-5-(2-chloro-5-méthylphényl)pyridin-2-yl]carbonyl}amino)-4-méthylpentanoique

Selon la séquence saponification/couplage peptidique décrite dans les exemples 1.7 et 9 respectivement, on obtient, à partir de 385 mg (0,84 mmoles) de 6-{4-chloro-3-[3-(diméthylamino)propoxy]phényl}-5-(2-chloro-5-méthylphényl)pyridine-2-carboxylate de méthyle et 130 mg (1 mmoles) d'acide (3S)-3-amino-4-méthylpentanoique (J. Org. Chem., 1999, 6411-6417), 333 mg de chlorhydrate d'acide (3S)-3-({[6-{4-chloro-3-[3-(diméthylamino)propoxy]phényl}-5-(2-chloro-5-méthylphényl)pyridin-2-yl]carbonyl}amino)-4-méthylpentanoique sous forme de poudre blanche.
Rendement = 67 %
F(°C) = 157
[α]_{D}²² = -18° ; (c = 0.1 ; MeOH)
M = C₃₀H₃₅Cl₂N₃O₄ = 571 ; M+H = 572
RMN1H (ppm, d6-DMSO, 400 MHz) : 8,60 (dd, 1H) ; 8,10 (d, 1 H) ; 7.95 (d, 1 H) ; 7,40-7,15 (m, 5H) ; 6,90 (d, 1H) ; 4,15 (m, 1H) ; 3,90 (m, 2H) ; 3,15 (t, 2H) ; 2,75 (s, 6H) ; 2,55 (t, 2H) ; 2,25 (d, 3H (cooformères) ; 2,10 (m, 2H) ; 1,90 (m, 1H) ; 0,85 (dd, 6H (conformères)).

### Exemple 15 : Chlorhydrate d'acide 1-({[6-{4-chloro-3-[3-(diméthylamino)propoxyl]phényl}-5-(2,4-diméthylphényl)pyridin-2-yl]carbonyl}amino)cyclohexanecarboxylique (composé N°66)

### 15.1. 6-[3-(benzyloxy)-4-chlorophényl]-5-(2,4-diméthylphényl)pyridine-2-carboxylate de méthyle

Selon le procédé décrit dans l'exemple 4.5, le couplage de Suzuki effectué entre 1 g (2 mmoles) de 6-[3-(benzyloxy)-4-chlorophényl]-5-([(trifluorométhyl)sulfonyl]oxy}pyridine-2-carboxylate de méthyle et 418 mg (2,8 mmoles) d'acide 2,4-diméthylphénylboronique conduit à 820 mg de 6-[3-(benzyloxy)-4-chlorophényl]-5-(2,4-diméthylphényl)pyridine-2-carboxylate de méthyle sous forme d'huile.
Rendement = 90 %

### 15.2. Chlorhydrate d'acide 6-{4-chloro-3-[3-(diméthylamino)propoxy]phényl}-5-(2,4-diméthylphényl)pyridine-2-carboxylique

Selon la séquence débenzylation/O-alkylation/saponification décrite dans les exemples 4.6, 1.6 et 1.7 respectivement, on obtient à partir de 820 mg (1,8 mmoles) de 6-[3-(benzyloxy)-4-chlorophényl]-5-(2,4-diméthylphényl)pyridine-2-carboxylate de méthyle, 659 mg de chlorhydrate d'acide 6-{4-chloro-3-[3-(diméthylamino)propoxylphényl}-5-(2,4-diméthylphényl)pyridine-2-carboxylique sous forme de gomme.
Rendement = 77 %

### 15.3. Chlorhydrate d'acide 1-({[6-{4-chloro-3-[3-(diméthylamino)propoxy]phényl}-5-(2,4-diméthylphényl)pyridin-2-yl]carbonyl}amino)cyclohexanecarboxylique

Selon le procédé décrit dans l'exemple 1.8, le couplage peptidique effectué entre 356 mg (0,75 mmoles) de chlorhydrate d'acide 6-{4-chloro-3-[3-(diméthylamino)propoxy]phényl}-5-(2,4-diméthylphényl)pyridine-2-carboxylique et 113 mg (0,79 mmoles) d'acide 2-aminocyclohexane-2-carboxylique conduit à 160 mg de chlorhydrate d'acide 1-({[6-{4-chloro-3-[3-(diméthylamino)propoxy]phényl}-5-(2,4-diméthylphényl)pyridin-2-yl]carbonyl}amino)cyclohexanecarboxylique sous forme de poudre blanche.
Rendement = 38 %
F(°C) = 158
M = C₃₂H₃₆ClN₃O₄ = 563. ; M+H = 564
RMN¹H (ppm, d6-DMSO, 400 MHz) : 12,45 (s, 1H) ; 10,40 (s, 1 H) ; 8.45 (s, 1H) ; 8,05 (d, 1H) ; 7,95 (d, 1 H) ; 7,40 (d, 1 H) ; 7,20 (s, 1 H) ; 7,15 (s, 2H) ; 2,75 (s, 6H) ; 7,10 (d, 1H) ; 7,05 (d, 1H) ; 3,90 (m, 2H) ; 3,20 (t, 2H) ; 2,70 (s, 6H) ; 2,35 (s, 3H) ; 2,2-2,05 (m, 4H) ; 1,90 (s, 3H) ; 1,85 (t, 2H) ; 1,75-1,30 (m, 6H).

### Exemple N°16 : Chlorhydrate d'acide (3S)-3-({[6-{4-chloro-3-[3-(diméthylamino)propoxy]phényl}-5-(2,4-diméthylphényl)pyridin-2-yl]carbonyl}amino)-4,4-diméthylpentanoique. (composé N°117)

Selon le procédé décrit dans l'exemple 9, le couplage peptidique effectué entre 950 mg (2 mmoles) de chlorhydrate d'acide 6-{4-chloro-3-[3-(diméthylamino)propoxy]phényl}-5-(2,4-diméthylphényl)pyridine-2-carboxylique et 412 mg (2,05 mmoles) de (3S)-3-amino-4,4-diméthylpentanoate de tert-butyle (J.Org-Chem., 1999, 64, 6411-6417) conduit à 830 mg de chlorhydrate d'acide (3*S*)-3-({[6-{4-chloro-3-[3-(diméthylamino)propoxy]phényl}-5-(2,4-diméthylphényl)pyidin-2-yl]carbonyl}amino)-4,4-diméthylpentanoique sous forme de poudre blanche.
Rendement = 70%
F(°C) = 130
[α]_{D}²² = -5° ; (c = 0,1 ; MeOH)
M = C₃₂H₄₀ClN₃O₄ = 565 ; M+H = 566
RMN¹H (ppm, d6-DMSO, 400 MHz) : 10,09 (s, 1H) ; 8.2 (d, 1H) ; 7,85 (d, 1H) ; 7,70 (d, 1H) ; 7,10 (d, 1H) ; 6,95 (d, 1H) ; 6,85 (m, 3H) ; 6,80 (t, 1H) ; 4,10 (t, 1H) ; 3,65 (m, 2H) ; 2,95 (m, 2H) ; 2,50 (d, 6H) ; 2,40 (dd, 1H) ; 2,30 (dd, 1H) ; 2,10 (s, 3H) ; 1,85 (m, 2H) ; 1,65 (s, 3H) ; 0.70 (d, 9H, conformères).

### Exemple N°17 Chlorhydrate d'acide 1-{[(3,5-dichloro-2'-{4-chloro-3-[3-(diméthylamino)propoxy]phényl}-2,3'-bipyridin-6'-yl)carbonyl]amino}cyclohexanecarboxylique (composé N°65)

### 17.1. 2'-[3-(benzyloxy)-4-chlorophényl]-3,5-dichloro-2,3'-bipyridine-6'-carboxylate de méthyle

Selon le procédé décrit dans l'exemple 13.2, le couplage de Suzuki-Myaura effectué entre 4,4 g (9,17 mmoles) de 6-[3-(benzyloxy)-4-chlorophényl]-5-(4,4,5,5-tétraméthyl-1,3,2-dioxaborolan-2-yl}pyridine-2-carboxylate de méthyle et 2,5 g (11 mmoles) de 2-bromo-3,5-dichloropyridine conduit à 3 g de 2'-[3-(benzyloxy)-4-chlorophényl]-3,5-dichloro-2,3'-bipyridine-6'-carboxylate de méthyle sous forme d'huile.
Rendement = 65 %

### 17.2. 3,5-dichloro-2'-{4-chloro-3-[3-(diméthylamino)propoxy]phényl}-2,3'-bipyridine-6'-carboxylate de méthyle

Selon la séquence débenzylation/O-alkylation décrite dans les exemples 4.6 et 1.6 respectivement, on obtient à partir de 3 g (6,2 mmoles) de 2'-[3-(benzyloxy)-4-chlorophényl]-3,5-dichloro-2,3'-bipyridine-6'-carboxylate de méthyle, 1,8 g de 3,5-dichloro-2'-{4-chloro-3-[3-(diméthylamino)propoxy]phényl}-2,3'-bipyridine-6'-carboxylate de méthyle sous forme de gomme.
Rendement = 58 %

### 17.3. Chlorhydrate d'acide 1-{[(3,5-dichloro-2'-{4-chloro-3-[3-(diméthylamino)propoxy]phényl}-2,3'-bipyridin-6'-yl)carbonyl]amino}cyclohexanecarboxylique

Selon la séquence saponification / couplage peptidique décrite dans les exemples 1.7 et 1.8 respectivement, on obtient, à partir de 1,8 g (3,62 mmoles) de 3,5-dichloro-2'-{4-chloro-3-[3-(diméthylamino)propoxy]phéenyl}-2,3'-bipyridine-6'-carboxylate de méthyle et 472 mg (3,3 mmoles) d'acide 2-aminocyclohexane-2-carboxylique, 1,12 g de chlorhydrate d'acide 1-{[(3,5-dichloro-2'-{4-chloro-3-[3-(diméthylamino)propoxy]phényl}-2,3'-bipyridin-6'-yl)carbonyl]amino}cyclohexanecarboxylique sous forme de poudre blanche.
Rendement = 50 %
F(°C) = 165
M C₂₉H₃₁Cl₃N₄O₄ = 604 ; M+H = 605
RMN¹H (ppm, d6-DMSO, 400 MHz) : 8,75 (s, 1H) ; 8.45 (s, 1H) ; 8,30 (d, 1H) ; 8,15 (dd, 2H) ; 7,35 (d, 1H) ; 7,30 (s, 1 H) ; 6,75 (dd, 1H) ; 4,00 (t, 2H) ; 3,20 (m, 2H) ; 2,75 (d, 6H) ; 2,3-1,3 (m, 12H).

### Exemple N°18 : Chlorhydrate d'acide 1-({[6-{4-chloro-3-[3-(diméthylamino)propoxy]phényl}-5-(3,5-diméthyl-1H-pyrazol-1-yl)pyridin-2-yl]carbonyl}amino)cyclohexanecarboxylique (composé N°75)

### 18.1. 6-[3-(benzyloxy)-4-chlorophényl]-5-[2-(diphénylméthylidène)hydrazino]pyridine-2-carboxylate de méthyle

Dans un tube à vis, on introduit 2 g (4 mmoles) de 6-[3-(benzyloxy)-4-chlorophényl]-5-{[(trifluorométhyl)sulfonyl]oxy}pyridine-2-carboxylate de méthyle dans 10 mL de toluène anhydre. A la solution placée sous argon, sont ajoutés successivement 938 mg (4,78 mmoles) de benzophénone hydrazone, 1,95 g (6 mmoles) de carbonate de césium et 65 mg (0,08 mmoles) de PdCl₂ (dppf). Le mélange réactionnel est placé 3 h à 90°C sous agitation puis réparti dans 100 mL d'un mélange éther/eau 1 :1. La phase organique est séchée sur Na₂SO₄ et concentrée sous pression réduite. Le résidu obtenu est purifié par chromatographie sur une colonne de gel de silice, en éluant avec un gradient DCM/acétone de 0 à 5 % en acétone. Après concentration sous pression réduite, on obtient 1,94 g de 6-[3-(benzyloxy)-4-chlorophényl]-5-[2-(diphénylméthylidéne)hydrazino]pyridine-2-carboxylate de méthyle sous forme d'huile.
Rendement = 88 %

### 18.2. 6-[3-(benzyloxy)-4-chlorophényl]-5-(3,5-diméthyl-1H-pyrazol-1-yl)pyridine-2-carboxylate d'éthyle

Dans un tube à vis on introduit 600 mg (1,08 mmoles) de 6-[3-(benzyloxy)-4-chlorophényl]-5-[2-(diphénylméthylidène)hydrazino]pyridine-2-carboxylate de méthyle, 378 mg (3,78 mmoles) de 2,4-pentanedione, 186 mg (1,08 mmoles) d'acide paratoluènesulfonique et 5 mL d'éthanol. Le mélange est chauffé à 120°C sous agitation pendant 48 h, refroidi puis concentré sous pression réduite. Le résidu est ensuite réparti dans 50 mL d'AcOEt et 50 mL d'une solution aqueuse saturée en NaHCO₃. La phase organique est séchée sur Na₂SO₄ et concentrée sous pression réduite. Le résidu obtenu est purifié par chromatographie sur une colonne de gel de silice en éluant avec un gradient heptane/AcOEt de 0 à 50 % en AcOEt. Après concentration sous pression réduite, on obtient 235 mg de 6-[3(benzyloxy)-4-chlorophényl]-5-(3,5-diméthyl-1*H-*pyrazol-1-yl)pyridine-2-carboxylate d'éthyle sous forme d'huile.
Rendement = 47 %

### 18.3. Chlorhydrate d'acide 6-{4-chloro-3-[3-(diméthylamino)propoxy]phényl}-5-(3,5-diméthyl-1H-pyrazol-1-yl)pyridine-2-carboxylique

Selon la séquence débenzylation /O-alkylation / saponification décrite dans les exemples 4.6, 1.6 et 1.7 respectivement, on obtient à partir de 1 g (2,25 mmoles) de 6-[3-(benzyloxy)-4-chlorophényl]-5-(3,5-diméthyl-1*H*-pyrazol-1-yl)pyridine-2-carboxylate d'éthyle, 460 mg de chlorhydrate d'acide 6-{4-chloro-3-[3-{diméthylamino)propoxy]phényl}-5-(3,5-diméthyl-1*H*-pyrazol-1-yl)pyridine-2-carboxylique.
Rendement = 43 %

### 18.4. Chlorhydrate d'acide 1-({[6-{4-chloro-3-[3-(diméthylamino)propoxy]phényl}-5-(3,5-diméthyl-1H-pyrazol-1-yl)pyridin-2-yl]carbonyl}amino)cyclohexanecarboxylique

Selon le procédé décrit dans l'exemple 9, le couplage peptidique effectué entre 460 mg (1 mmole) de chlorhydrate d'acide 6-{4-chloro-3-[3-(diméthylamino)propoxy]phényl}-5-(3,5-diméthyl-1*H*-pyrazol-1-yl)pyridine-2-carboxylique et 156 mg (1,09 mmoles) d'acide 2-aminocyclohexane-2-carboxylique conduit à 270 mg de chlorhydrate d'acide 1-({[6-{4-chloro-3-[3-(diméthylamino)propoxy]phényl}-5-(3,5-diméthyl-1*H*-pyrazol-1-yl)pyridin-2-yl]carbonyl}amino)cyclohexanecarboxylique sous forme de poudre blanche.
Rendement = 46 %
F(°C) = 202
M C₂₉H₃₆ClN₅O₄ = 553 ; M+H = 554
RMN¹H (ppm, d6-DMSO, 400 MHz) : 12,45 (s, 1H) ; 10,3 (s, 1H) ; 8,40 (s, 1H) ; 8.15 (s, 2H) ; 7,45 (d, 1H) ; 7,00 (m, 2H) ; 6,05 (s, 1 H) ; 3,95 (t, 2H) ; 3,15 (m, 2H) ; 2,75 (d, 6H) ; 2,20 (s, 3H) ; 2,15 (m, 4H) ; 1,70 (s, 3H) ; 2,9-1,2 (m, 8H).

### Exemple N°19 Chlorhydrate d'acide 1-({[6-{4-chloro-3-[3-(diméthylamino)propoxy]phényl}-5-(2-méthylphényl)pyrazin-2-yl]carbonyl}amino)cyclohexanecarboxylique (composé N°80)

### 19.1. 5-aminopyrazine-2-carbonitrile

Une suspension de 1,85 g (20,7 mmoles) de cyanure de cuivre et de1 g (20,7 mmoles) de cyanure de sodium dans 20 ml de DMF est portée sous agitation à 135°C. A la solution obtenue sont ajoutés 3,6 g (20,7 mmoles) de 5-bromopyrazin-2-amine et la température de 135°C est maintenue pendant 18 h. On rajoute alors 2 équivalents de cyanure de sodium et de cyanure de cuivre et le chauffage est maintenu pendant encore 24 h. Après refroidissement, on ajoute 100 mL d'une solution aqueuse 0,3 N de cyanure de sodium, on agite le mélange 1 h à 40°C puis on le répartit dans 300 mL d'un mélange AcOEt / eau 1:1. Après lavage avec 2 x 100 mL d'eau, la phase organique est séchée sur Na₂SO₄ et concentrée sous pression réduite. Le résidu obtenu est purifié par chromatographie sur une colonne de gel de silice en éluant avec un gradient cyclohexane/AcOEt de 0 à 100 % en AcOEt. Après concentration sous pression réduite, on obtient 1,24 g de 5-aminopyrazine-2-carbonitrile sous forme d'huile.
Rendement = 50 %

### 19.2. 5-aminopyrazine-2-carboxylate de méthyle

Une solution de 2,24 g (18,65 mmoles) de 5-aminopyrazine-2-carbonitrile et de 9,45 mL (74,40 mmoles) d'éthérate de trifluorure de bore dans 50 mL de méthanol est portée 2 h à reflux. Le mélange réactionnel est concentré sous pression réduite et le résidu obtenu est repris par 200 mL d'AcOEt et 10 mL d'une solution aqueuse saturée en NaHCO₃. La phase organique est séchée sur Na₂SO₄ et concentrée sous pression réduite. Le résidu obtenu est purifié par chromatographie sur une colonne de gel de silice en éluant avec un mélange cyclohexane/AcOEt 1:1. Après concentration sous pression réduite, on obtient 1,4 g de 5-aminopyrazine-2-carboxylate de méthyle sous forme d'huile.
Rendement = 49 %

### 19.3. 5-amino-6-bromopyrazine-2-carboxylate de méthyle

A une solution de 1,4 g (9,14 mmoles) de 5-aminopyrazine-2-carboxylate de méthyle dans 10 mL d'acétonitrile sont ajoutés 1,79 g (10,05 mmoles) de N-bromosuccinimide. Le mélange réactionnel est agité 2 h à 20°C puis réparti dans 100 mL d'un mélange AcOEt / eau 1:1. La phase organique est lavée avec 2x50 mL d'eau, séchée sur Na₂SO₄ et concentrée sous pression réduite. Le résidu obtenu est purifié par chromatographie sur une colonne de gel de silice en éluant avec un gradient cyclohexane / AcOEt de 0 à 50 % en AcOEt. Après concentrations sous pression réduite, on obtient 1,66 g de 5-amino-6-bromopyrazine-2-carboxylate de méthyle sous forme de cire jaune.
Rendement = 78 %

### 19.4. 5-amino-6-[3-(benzyloxy)-4-chlorophényl]pyrazine-2-carboxylate de méthyle

Une suspension de 137 mg (0,36 mmoles) de dichlorure de (bis-benzonitrile)-palladium **II** et de 183 mg (0,43 mmoles) de diphénylphosphinobutane dans 7 mL de toluène sous argon est agitée 30 min à température ambiante. On ajoute ensuite sous barbotage d'argon 1,66 g (7,15 mmoles) de 5-amino-6-bromopyrazine-2-carboxylate de méthyle, 1,97 g (7,51 mmoles) d'acide [3-(benzyloxy)-4-chlorophényl]boronique, 2,4 mL d'éthanol et 3,58 mL (7,15 mmoles) d'une solution aqueuse 2N de carbonate de sodium. Le milieu réactionnel est porté 5h30 à reflux puis réparti dans 100 mL d'un mélange eau/AcOEt 1:1. La phase organique est lavée avec 50 mL d'eau, séchée sur Na₂SO₄ et concentrée sous pression réduite. Le résidu obtenu est purifié par chromatographie sur une colonne de gel de silice en éluant avec un gradient cyclohexane/AcOEt de 50 à 100 % d'AcOEt. Après concentration sous pression réduite, on obtient 1,33 g de 5-amino-6-[3-(benzyloxy)-4-chlorophényl]pyrazine-2-carboxylate de méthyle sous forme de cire jaune.
Rendement = 50 %

### 19.5. 6-[3-(bonzyloxy)-4-chlorophényl]-5-bromopyrazine-2-carboxylate de méthyle

A une solution de 1,28 g (3,46 mmoles) de 5-amino-6-[3-(benzyloxy)-4-chlorophényl]pyrazine-2-carboxylate de méthyle dans 10 mL d'acétonitrile anhydre refroidie à 0°C sous argon sont ajoutés goutte à goutte 0,82 mL (6,9 mmoles) de nitrite de tert-butyle. Après 2 h à 0°C, on ajoute 1,54 g de dibromure de cuivre et la suspension obtenue est agitée 1h30 à 65°C. Après refroidissement, le mélange réactionnel est réparti dans 100 mL d'un mélange AcOEt/eau 1:1. La phase organique est lavée avec 3 x 50 mL d'eau et 50 mL de saumure puis est séchée sur Na₂SO₄ et concentrée sous pression réduite. Le résidu obtenu est purifié par chromatographie sur une colonne de gel de silice en éluant avec un gradient cyclohexane / AcOEt de 0 à 30 % en AcOEt. Après concentration sous pression réduite, on obtient 645 mg de 6-[3-(benzyloxy)-4-chlorophényl]-5-bromopyrazine-2-carboxylate de méthyle sous forme de cire jaune.
Rendement = 43 %

### 19.6. 6-[3-(benzyloxy)-4-chlorophényl]-5-(2-méthylphényl)pyrazine-2-carboxylate de méthyle

Selon le procédé décrit dans l'exemple 4.5, le couplage de Suzuki effectué entre 545 mg (1,26 mmoles) de 6-[3-(benzyloxy)-4-chlorophényl]-5-bromopyrazine-2-carboxylate de méthyle et 205 mg (1,51 mmoles) d'acide 2-méthylphénylboronique conduit à 430 mg de 6-[3-(benzyloxy)-4-chlorophényl]-5-(2-méthylphényl)pyrazine-2-carboxylate de méthyle sous forme de cire.
Rendement = 76 %

### 19.7. Chlorhydrate d'acide 1-({[6-{4-chloro-3-[3-(diméthylamino)propoxy]phényl}-5-(2-méthylphényl)pyrazin-2-yl]carbonyl}amino)cyclohexanecarboxylique

Selon la séquence débenzylation / O-alkylation / saponification / couplage peptidique décrite dans les exemples 4.6, 1.6, 1.7 et 9, on obtient à partir de 485 mg (1,09 moles) de 6-[3-(benzyloxy)-4-chlorophényl]-5-(2-méthylphényl)pyrazine-2-carboxylate de méthyle, 154 mg de chlorhydrate d'acide 1-({[6-{4-chloro-3-[3-(diméthylamino)propoxy]phényl}-5-(2-méthylphényl)pyrazin-2-yl]carbonyl}amino)cyclohexanecarboxylique sous forme de poudre blanche.
Rendement = 20 %
F(°C) = 162
M C₃₀H₃₅ClN₄O₄ = 550. ; M+H = 551
RMN¹H (ppm, d6-DMSO, 400 MHz) : 10,40 (s, 1H) ; 9,15 (s, 1 H) ; 8.40 (s, 1H) ; 7,40 (d, 1H) ; 7,30 (m, 5H) ; 7,10 (dd, 1H) ; 3,85 (t, 2H) ; 3,15 (m, 2H) ; 2,75 (d, 6H) ; 1,95 (s, 3H) ; 2,3-1,3 (m, 12H).

### Exemple N°20 Chlorhydrate d'acide 1-({[3-amino-6-{4-chloro-3-[3-(diméthylamino)propoxy]phényl}-5-(2-méthylphényl)pyrazin-2-yl]carbonyl}amino)cyclohexanecarboxylique (composé N°129)

### 20.1. 3-amino-6-chloro-5-(2-méthylphényl)pyrazine-2-carboxylate de méthyle

Dans un tube à vis, on introduit 5 g (22,5 mmoles) de 3-amino-5,6-dichloropyrazine-2-carboxylate de méthyle, 3,2 g. (23,65 mmoles) d'acide 2-méthylphényl boronique dans 45 mL de toluène anhydre. Après solubilisation, on ajoute 34 mL (67,6 mmoles) d'une solution aqueuse 2N de carbonate de sodium et le mélange biphasique est dégazé 30 min par barbotage d'argon. On ajoute alors 1,3 g (1,13 mmoles) de Pd(PPh₃)₄ et le mélange réactionnel est agité vigoureusement à 110°C pendant 48 h. Après refroidissement, la solution est répartie dans 500 mL d'un mélange AcOEt/saumure 1:1 et la phase aqueuse est re-extraite par 4 x 50 mL d'AcOEt. Les phases organiques sont réunies, séchées sur Na₂SO₄ et concentrées sous pression réduite. Le résidu obtenu est purifié par chromatographie sur une colonne de gel de silice en éluant avec un gradient cyclohexane/AcOEt de 0 à 20 % en AcOEt. Après concentration sous pression réduite, on obtient 2 g de 3-amino-6-chloro-5-(2-méthylphényl)pyrazine-2-carboxylate de méthyle sous forme de cire jaune.
Rendement 50 %

### 20.2. 3-amino-6-[3-(benzyloxy)-4-chlorophényl]-5-(2-méthylphényl)pyrazine-2-carboxylate de méthyle

Selon le procédé décrit précédemment (exemple 20.1), le couplage de Suzuki effectué entre 1,7 g (6,12 mmoles) de 3-amino-6-chloro-5-(2-méthylphényl)pyrazine-2-carboxylate de méthyle et 3,2 g (12,24 mmoles) de 5-amino-6-bromopyrazine-2-carboxylate de méthyle, 1,97 g (7,51 mmoles) d'acide [3-(benzyloxy)-4-chlorophényl]boronique conduit à 2,7 g de 3-amino-6-[3-(benzyloxy)-4-chlorophényl]-5-(2-méthylphényl)pyrazine-2-carboxylate de méthyle sous forme de gomme.
Rendement = 80 %

### 20.3. Chlorhydrate d'acide 1-({[3-amino-6-{4-chloro-3-[3-(diméthylamino)propoxy]phényl}-5-(2-méthylphényl)pyrazin-2-yl]carbonyl}amino)cyclohexanecarboxylique

Selon la séquence débenzylation / O-alkylation / saponification / couplage peptidique décrite dans les exemples 4.6, 1.6, 1.7 et 9, on obtient à partir de 263 mg (0,57 mmoles) de 3-amino-6-[3-(benzyloxy)-4-chlorophényl]-5-(2-méthylphényl)pyrazine-2-carboxylate de méthyle, 40 mg de chlorhydrate d'acide 1-({[3-amino-6-{4-chloro-3-[3-(diméthylamino)propoxy]phényl}-5-(2-méthylphényl)pyrazin-2-yl]carbonyl}amino)cyclohexanecarboxylique sous forme de poudre blanche.
Rendement = 12,5 %
F(°C) = 138
M C₃₀H₃₆ClN₅O₄ = 565 ; M+H = 566
RMN¹H (ppm, d6-DMSO, 400 MHz) : 12,40 (s, 1H) ; 10,1 (s, 1H) ; 8.30 (s, 1H) ; 7,55 (s, 2H) ; 7,30 (q, 1H) ; 7,20 (m, 4H) ; 7,05 (s, 1 H) ; 6,85 (d, 1 H) ; 3,80 (t, 2H) ; 3,10 (m, 2H) ; 2,70 (s, 6H) ; 2,15 (d, 2H) ; 2,05 (m, 2H) : 1,95 (s, 3H) ; 1,75 (t, 2H) ; 1,55 (m, 3H) ; 1,40 (m, 2H) ; 1,25 (m, 1H).

### Exemple N°21 : Chlorhydrate d'acide 1-({[6-{4-chloro-3-[3-(diméthylamino)propoxy]phényl}-5-(2-chloro-5-éthoxyphényl)pyridin-2-yl]carbonyl}amino)cyclohexanecarboxylique (composé N°105)

### 21.1. 4-chloro-3-iodophénol

Selon la séquence réduction / réaction de Sandmeyer décrite dans les exemples 12.4 et 12.1 respectivement, on obtient à partir de 25 g (144 mmoles) de 4-chloro-3-nitrophénol 24 g de 4-chloro-3-iodophénol sous forme d'huile.
Rendement = 66 %

### 21.2. 1-chloro-2-iodo-4-éthoxybenzène

A une solution de 2,45 g (9,6 mmoles) de 4-chloro-3-iodophénol dans 30 mL de DMF sont ajoutés 2 g (14,5 mmoles) de K₂CO₃ et 1,16 mL (14,5 mmoles) de iodoéthane. Le milieu réactionnel est chauffé à 50°C sous agitation pendant 3 h puis réparti dans 200 mL d'un mélange éther/eau 1:1. La phase organique est lavée avec 3x50 mL d'eau puis séchée sur Na₂SO₄ et concentre sous pression réduite. On obtient 2,38 g de 1-chloro-2-iodo-4-éthoxybenzène sous forme d'huile.
Rendement = 87 %

### 21.3. Acide (2-chloro-5-éthoxyphényl)boronique

A une solution de 2,38 g (8,4 mmoles) de 1-chloro-2-iodo-4-éthoxybenzène dans 50 mL de THF anhydre refroidie sous argon à -78°C, sont ajoutés goutte à goutte 5,5 mL (8,8 mmoles) d'une solution 1,6 N de butyllithium dans l'hexane pendant 30 min. Après 2 h à -70°C, on ajoute 1,74 g (9,2 mmoles) de borate de triisopropyle et le milieu réactionnel est agité 3 h à température ambiante puis on réparti dans 200 mL d'un mélange AcOEt/HCl aq 5N 1:1. La phase organique est lavée avec 50 mL d'eau, séchée sur Na₂SO₄ et concentrée sous pression réduite. Le résidu obtenu est purifié par chromatographie sur une colonne de gel de silice en éluant avec un gradient DCM/méthanol de 0 à 10 % en méthanol. Après concentration sous pression réduite, on obtient 990 mg d'acide (2-chloro-5-éthoxyphényl)boronique, sous forme d'huile.
Rendement = 59 %

### 21.4. 5-(2-chloro-5-éthoxyphényl)-6-{4-chloro-3-[(4-méthoxybenzyl)oxy]phényl}pyridine-2-carboxylate de méthyle

Selon le procédé décrit dans l'exemple 4.5, la réaction de Suzuki effectuée entre 452 mg (2,26 mmoles) d'acide (2-chloro-5-éthoxyphényl)boronique et 1 g (1,88 mmoles) de 6-{4-chloro-3-[(4-méthoxybenzyl)oxy]phényl}-5-{[(rifluorométhyl)sulfonyl]oxy}pyridine-2-carboxylate de méthyle (obtenu en 3 étapes à partir de 1-chloro-4-iodo-2-[(4-méthoxybenzyl)oxy]benzène selon la séquence borylation / couplage de Suzuki / triflate décrite dans les exemples 4.2, 4.3 et 4.4 respectivement) conduit à 700 mg de 5-(2-chloro-5-éthoxyphényl)-6-{4-chloro-3-[(4-méthoxybenzyl)oxy]phényl}pyridine-2-carboxylate de méthyle sous forme d'huile.
Rendement = 70 %

### 21.5. 5-(2-chloro-5-éthoxyphényl)-6-(4-chloro-3-hydroxyphényl)pyridine-2-carboxylate de méthyle

A une solution de 700 mg (1,3 mmoles) de 5-(2-chloro-5-éthoxyphényl)-6-{4-chloro-3-[(4-méthoxybenzyl)oxy]phényl}pyridine-2-carboxylate de méthyle dans 10 mL de DCM agitée à 0°C sont ajoutés 1 mL (13 mmoles) d'acide trifluoroacétique. Le mélange réactionnel est agité 2 h à 20°C puis concentré sous pression réduite. Le résidu est repris par 50 mL de DCM et lavé avec 50 mL d'une solution aqueuse saturée en NaHCO₃. La phase organique est séchée sur Na₂SO₄ et concentrée sous pression réduite. Le résidu obtenu est purifié par chromatographie sur une colonne de gel de silice en éluant avec un gradient cyclohexane / AcOEt de 0 à 30 % en AcOEt. Après concentration sous pression réduite, on obtient 524 mg de 5-(2-chloro-5-éthoxyphényl)-6-(4-chloro-3-hydroxyphényl)pyridine-2-carboxylate de méthyle sous forme d'huile.
Rendement = 96 %

### 21.6. Chlorhydrate d'acide 1-({[6-{4-chloro-3-[3-(diméthylamino)propoxy]phényl}-5-(2-chloro-5-éthoxyphényl)pyridin-2-yl]carbonyl}amino)cyclohexanecarboxylique

Selon la séquence O-alkylation / saponification / couplage peptidique décrite dans les exemples 1.6, 1.7 et 9, on obtient à partir de 524 mg (1,25 mmoles) de 6-{4-chloro-3-[(4-méthoxybenzyl)oxy]phényl}-5-{[(trifluorométhyl)sulfonyl]oxy}pyridine-2-carboxylate de méthyle, 125 mg de chlorhydrate d'acide 1-({[6-{4-chloro-3-[3-(diméthylamino)propoxy]phényl}-5-(2-chloro-5-éthoxyphényl)pyridin-2-yl]carbonyl}amino)cyclohexanecarboxylique sous forme de poudre blanche.
Rendement = 18 %
F(°C) = 160
M C₃₂H₃₇Cl₂N₃O₅ = 613 ; M+H = 614
RMN¹H (ppm, d6-DMSO, 400 MHz) : 8,45 (s, 1H) ; 8,10 (d, 1H) ; 8,00 (d, 1H) ; 7,40 (d, 2H) ; 7,35 (s, 1H) ; 7,00 (m, 3H) ; 4,05 (m, 2H) ; 3,95 (m, 2H) ; 3,20 (m, 2H) ; 2,80 (s, 6H) ; 2,20 (d, 2H) ; 2,15 (m, 2H) ; 1,85 (t, 2H) ; 1,65 (m, 3H) ; 1,45 (m, 2H) ; 1,35 (m, 1H) ; 1,30 (t, 3H).

## Revendications

1. Composés répondant à la formule (IV) : 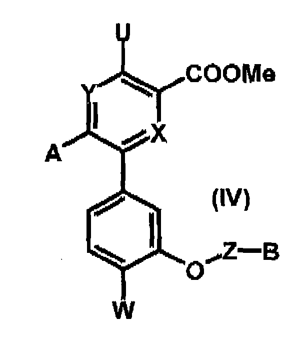 dans laquelle :
X et Y représentent indépendamment l'un de l'autre un atome d'azote ou un chaînon -CR3-, où R3 représente un atome d'hydrogène ou d'halogène ou un groupe alkyle ou alcoxy ;
U représente un atome d'hydrogène ou un groupe NHR7 où R7 est un atome d'hydrogène ou un groupe alkyle ;
A représente un groupe aryle, hétéroaryle ou hétérocycloalkyle éventuellement substitué en des positions quelconques par un ou plusieurs groupes choisis parmi un atome d'halogène et les groupes cyano, alkyle, halogénoalkyle, hydroxy, alcoxy, -O-(CH₂)ₚ-O-alkyle, halogénoalcoxy, -NRR', -NR-CO-alkyle et -SO₂-alkyle, où R et R' représentent indépendamment l'un de l'autre un atome d'hydrogène ou un groupe alkyle et p est un nombre entier compris entre 1 et 5 et plus particulièrement entre 1 et 3 ;
W représente un atome d'halogène, un groupe alkyle ou un groupe halogénoalkyle ;
Z représente une liaison, un groupe cycloalkylène ou un groupe alkylène éventuellement substitué par un ou plusieurs groupes choisis parmi un atome d'halogène et les groupes alkyle, hydroxy et alcoxy ;
B représente :
• soit un groupe -NR4R5, où R4 et R5 représentent indépendamment l'un de l'autre un groupe alkyle, cycloalkyle, hydroxyalkyle ou fluoroalkyle, ou bien R4 et R5 forment, avec l'atome d'azote auquel ils sont rattachés, un cycle à 5 ou 6 chaînons, tel qu'un cycle pyrrolidinyle ou pipéridinyle, éventuellement substitué par un groupe alkyle,
• soit un hétérocycle de formule suivante : 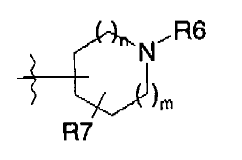
où m et n représentent indépendamment l'un de l'autre 0, 1 ou 2, et où R6 et R7 représentent indépendamment l'un de l'autre un atome d'hydrogène ou un groupe alkyle ou cycloalkyle ;
leurs sels, leurs énantiomères et diastéréoisomères, y compris leurs mélanges racémiques.

2. Composés selon la revendication 1, dans lesquels X et Y représentent indépendamment l'un de l'autre un atome d'azote ou un chaînon -CR3-, où R3 représente un atome d'hydrogène ou d'halogène ou un groupe alkyle ou alcoxy ;
leurs sels, leurs énantiomères et diastéréoisomères, y compris leurs mélanges racémiques.

3. Composés selon la revendication 1, dans lesquels U représente un atome d'hydrogène ou un groupe NHR7 où R7 est un atome d'hydrogène ou un groupe alkyle ;
leurs sels, leurs hydrates ou solvats ainsi que leurs énantiomères et diastéréoisomères, y compris leurs mélanges racémiques.

4. Composés selon la revendication 1, dans lesquels A représente un groupe aryle, hétéroaryle ou hétérocycloalkyle choisi parmi les groupes phényle, benzodioxolyle, thiényle, thiazolyle, pyridinyle, pyrimidinyle, pyridazinyle, pyrazinyle, pyrazolyle et pyrrolidinone, ledit groupe aryle, hétéroaryle ou hétérocycloalkyle étant éventuellement substitué en des positions quelconques par un ou plusieurs groupes choisis parmi un atome d'halogène et les groupes cyano, alkyle, halogénoalkyle, hydroxy, alcoxy, -O-(CH₂)ₚ-O-alkyle, halogénoalcoxy, -NRR', -NR-CO-alkyle, -SO- et -SO₂-alkyle, où R et R' représentent indépendamment l'un de l'autre un atome d'hydrogène ou un groupe alkyle et p est un nombre entier compris entre 1 et 5 ;
leurs sels, leurs hydrates ou solvats ainsi que leurs énantiomères et diastéréoisomères, y compris leurs mélanges racémiques.

5. Composés selon la revendication 1, dans lesquels W représente un atome d'halogène, un groupe alkyle ou un groupe halogénoalkyle ;
leurs sels, leurs énantiomères et diastéréoisomères, y compris leurs mélanges racémiques.

6. Composés selon la revendication 1, dans lesquels Z représente une liaison, un groupe cycloalkylène ou un groupe alkylène éventuellement substitué par un ou plusieurs groupes choisis parmi un atome d'halogène et les groupes alkyle, hydroxy et alcoxy ;
leurs sels, leurs énantiomères et diastéréoisomères, y compris leurs mélanges racémiques.

7. Composés selon la revendication 1, dans lesquels B représente un groupe -NR4R5, où R4 et R5 représentent indépendamment l'un de l'autre un groupe alkyle, cycloalkyle, hydroxyalkyle ou fluoroalkyle, ou bien R4 et R5 forment, avec l'atome d'azote auquel ils sont rattachés, un cycle à 5 ou 6 chaînons, tel qu'un cycle pyrrolidinyle ou pipéridinyle, éventuellement substitué par un groupe alkyle,
leurs sels, leurs énantiomères et diastéréoisomères, y compris leurs mélanges racémiques.

8. Composés selon la revendication 1, dans lesquels :
X et Y représentent indépendamment l'un de l'autre un atome d'azote ou un chaînon -CR3-, où R3 représente un atome d'hydrogène ou un groupe alcoxy ;
U représente un atome d'hydrogène ou un groupe NHR7 où R7 est un atome d'hydrogène ou un groupe alkyle ;
A représente un groupe aryle, hétéroaryle ou hétérocycloalkyle choisi parmi les groupes phényle, benzodioxolyle, thiényle, thiazolyle, pyridinyle, pyrazolyle et pyrrolidinone, ledit groupe aryle ou hétéroaryle étant éventuellement substitué en des positions quelconques par un ou plusieurs groupes choisis parmi un atome d'halogène et les groupes cyano, alkyle, halogénoalkyle, hydroxy, alcoxy, -O-(CH₂)ₚ-O-alkyle, halogénoalcoxy, -NRR', -NR-CO-alkyle et -SO₂-alkyle, où R et R' représentent indépendamment l'un de l'autre un atome d'hydrogène ou un groupe alkyle et p est un nombre entier compris entre 1 et 5 ;
W représente un atome d'halogène, un groupe alkyle ou un groupe halogénoalkyle ;
Z représente une liaison ou un groupe alkylène éventuellement substitué par au moins un groupe choisi parmi un atome d'halogène et les groupes alkyle et hydroxy ;
B représente :
• soit un groupe -NR4R5, où R4 et R5 représentent indépendamment l'un de l'autre un groupe alkyle, hydroxyalkyle, ou bien R4 et R5 forment, avec l'atome d'azote auquel ils sont rattachés, un cycle à 5 ou 6 chaînons, tel qu'un cycle pyrrolidinyle ou pipéridinyle,
• soit un hétérocycle de formule suivante : 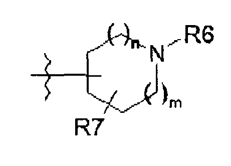
où m et n représentent indépendamment l'un de l'autre 0, 1 ou 2, et où R6 et R7 représentent indépendamment l'un de l'autre un atome d'hydrogène ou un groupe alkyle ou cycloalkyle ;
leurs sels, leurs énantiomères et diastéréoisomères, y compris leurs mélanges racémiques.

9. Composés selon la revendication 1, dans lesquels :
X et Y représentent indépendamment l'un de l'autre un atome d'azote ou un groupe CH ;
A représente un groupe phényle, pyridinyle, ou pyrrolidinone, substitué en des positions quelconques par 1 à 2 groupes choisis parmi un atome d'halogène, et les groupes alkyle, trifluorométhyle, méthoxy et N,N-diméthylamine ;
U représente un atome d'hydrogène ou un groupe NHR7 où R7 est un atome d'hydrogène ;
W représente un atome de chlore ou un groupe trifluorométhyle ;
Z représente une liaison ou un groupe alkylène éventuellement substitué par un groupe méthyle ;
B représente :
• soit un groupe -NR4R5, où R4 et R5 représentent indépendamment l'un de l'autre un groupe alkyle ou bien R4 et R5 forment, avec l'atome d'azote auquel ils sont rattachés, un cycle à 5 ou 6 chaînons,
• soit des hétérocycles de formule suivante : 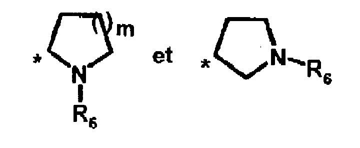
où m = 1 ou 2 et R6 représente un groupe éthyle ou méthyle;
leurs sels, leurs énantiomères et diastéréoisomères, y compris leurs mélanges racémiques.

10. Composés selon au moins l'une des revendications précédentes, dans lesquels :
X représente un atome d'azote et Y un groupe CH ;
A représente un groupe phényle ou pyridinyle, substitué dans les positions 2 ,4 ,5 et 6 par un ou deux groupes choisis parmi un atome d'halogène, tel que chlore ou fluor, et les groupes alkyles, tels que méthyle, éthyle ou isopropyle, trifluorométhyle, méthoxy et N,N-diméthylamine ;
U représente un atome d'hydrogène ou un groupe NHR7 où R7 est un atome d'hydrogène ;
W représente un atome de chlore ou un groupe trifluorométhyle ;
Z représente une liaison ou un groupe éthylène, propylène ou méthylpropylène ;
B représente :
• soit un groupe -NR4R5, où R4 et R5 représentent indépendamment l'un de l'autre un groupe méthyle, éthyle ou propyle ou forment ensemble avec l'atome d'azote auquel ils sont rattachés un cycle à 5 ou 6 chaînons,
• soit un hétérocycle de formule suivante : 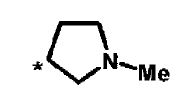
leurs sels, leurs hydrates ou solvats ainsi que leurs énantiomères et diastéréoisomères, y compris leurs mélanges racémiques.

11. Composés selon au moins l'une des revendications précédentes, dans lesquels :
X représente un atome d'azote et Y un groupe CH ;
A représente un groupe phényle ou pyridinyle, substitué dans les positions 2 ,4 ,5 et 6 par un ou deux groupes choisis parmi un atome d'halogène, tel que chlore ou fluor, et les groupes alkyles, tels que méthyle, éthyle ou isopropyle, trifluorométhyle, méthoxy et N,N-diméthylamine ;
U représente un atome d'hydrogène ou un groupe NHR7 où R7 est un atome d'hydrogéne ;
W représente un atome de chlore ou un groupe trifluorométhyle ;
Z représente une liaison ou un groupe éthylène, propylène ou méthylpropylène ;
B représente :
• un groupe -NR4R5, où R4 et R5 représentent indépendamment l'un de l'autre un groupe méthyle, éthyle ou propyle ou forment ensemble avec l'atome d'azote auquel ils sont rattachés un cycle à 5 ou 6 chaînons,
leurs sels, leurs hydrates ou solvats ainsi que leurs énantiomères et diastéréoisomères, y compris leurs mélanges racémiques.

12. Composé selon au moins l'une des revendications précédentes **caractérisé en ce qu'**il est un chlorhydrate.

## Patentansprüche

1. Verbindung mit der Formel (IV): 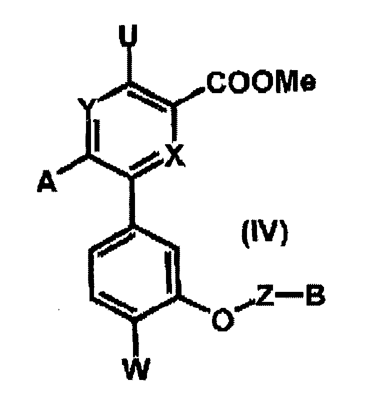 worin:
X und Y unabhängig voneinander für ein Stickstoffatom oder ein -CR3-Glied stehen, wobei R3 für ein Wasserstoff- oder Halogenatom oder eine Alkyl- oder Alkoxygruppe steht;
U für ein Wasserstoffatom oder eine NHR7-Gruppe steht, wobei R7 ein Wasserstoffatom oder eine Alkylgruppe ist;
A für eine Aryl-, Heteroaryl- oder Heterocycloalkylgruppe steht, die gegebenenfalls an beliebigen Positionen mit einer oder mehreren Gruppen, ausgewählt aus einem Halogenatom und den Cyano-, Alkyl-, Halogenalkyl-, Hydroxy-, Alkoxy-, -O-(CH₂)ₚ-O-Alkyl-, Halogenalkoxy-, -NRR'-, -NR-CO-Alkyl- und -SO₂-Alkylgruppen substituiert ist, wobei R und R' unabhängig voneinander für ein Wasserstoffatom oder eine Alkylgruppe stehen und p eine ganze Zahl im Bereich zwischen 1 und 5 und insbesondere im Bereich zwischen 1 und 3 ist;
W für ein Halogenatom, eine Alkylgruppe oder eine Halogenalkylgruppe steht;
Z für eine Bindung, eine Cycloalkylengruppe oder eine Alkylengruppe steht, die gegebenenfalls mit einer oder mehreren Gruppen, ausgewählt aus einem Halogenatom und den Alkyl-, Hydroxy- und Alkoxygruppen, substituiert ist;
B für Folgendes steht:
- entweder eine -NR4R5-Gruppe, wobei R4 und R5 unabhängig voneinander für eine Alkyl-, Cycloalkyl-, Hydroxyalkyl- oder Fluoralkylgruppe stehen, oder aber R4 und R5, mit dem Stickstoffatom, an das sie gebunden sind, einen 5- oder 6-gliedrigen Ring, wie etwa einen Pyrrolidinyl- oder Piperidinylring, bilden, der gegebenenfalls mit einer Alkylgruppe substituiert ist,
- oder einen Heterocyclus mit der folgenden Formel: 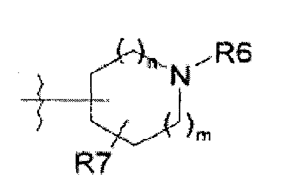
wobei m und n unabhängig voneinander für 0, 1 oder 2 stehen, und wobei R6 und R7 unabhängig voneinander für ein Wasserstoffatom oder eine Alkyl- oder Cycloalkylgruppe stehen;
deren Salzen, deren Enantiomeren und Diastereoisomeren, einschließlich deren razemischen Gemischen.

2. Verbindungen nach Anspruch 1, worin X und Y unabhängig voneinander für ein Stickstoffatom oder ein -CR3-Glied stehen, wobei R3 für ein Wasserstoff- oder Halogenatom oder eine Alkyl- oder Alkoxygruppe steht;
deren Salzen, deren Enantiomeren und Diastereoisomeren, einschließlich deren razemischen Gemischen.

3. Verbindungen nach Anspruch 1, worin U für ein Wasserstoffatom oder eine NHR7-Gruppe steht, wobei R7 ein Wasserstoffatom oder eine Alkylgruppe ist;
deren Salzen, deren Hydraten oder Solvaten sowie deren Enantiomeren und Diastereoisomeren, einschließlich deren razemischen Gemischen.

4. Verbindungen nach Anspruch 1, worin
A für eine Aryl-, Heteroaryl- oder Heterocycloalkylgruppe steht, ausgewählt aus den Phenyl-, Benzodioxolyl-, Thienyl-, Thiazolyl-, Pyridinyl-, Pyrimidinyl-, Pyridazinyl-, Pyrazinyl-, Pyrazolyl- und Pyrrolidinongruppen, wobei die Aryl-, Heteroaryl- oder Heterocycloalkylgruppe gegebenenfalls an beliebigen Positionen mit einer oder mehreren Gruppen, ausgewählt aus einem Halogenatom und den Cyano-, Alkyl-, Halogenalkyl-, Hydroxy-, Alkoxy-, -O-(CH₂)ₚ-O-Alkyl-, Halogenalkoxy-, -NRR'-, -NR-CO-Alkyl-und -SO₂-Alkylgruppen substituiert ist, wobei R und R' unabhängig voneinander für ein Wasserstoffatom oder eine Alkylgruppe stehen und p eine ganze Zahl im Bereich zwischen 1 und 5 ist;
deren Salzen, deren Hydraten oder Solvaten sowie deren Enantiomeren und Diastereoisomeren, einschließlich deren razemischen Gemischen.

5. Verbindungen nach Anspruch 1, worin W für ein Halogenatom, eine Alkylgruppe oder eine Halogenalkylgruppe steht;
deren Salzen, deren Enantiomeren und Diastereoisomeren, einschließlich deren razemischen Gemischen.

6. Verbindungen nach Anspruch 1, worin Z für eine Bindung, eine Cycloalkylengruppe oder eine Alkylengruppe steht, die gegebenenfalls mit einer oder mehreren Gruppen, ausgewählt aus einem Halogenatom und den Alkyl-, Hydroxy- und Alkoxygruppen, substituiert ist;
deren Salzen, deren Enantiomeren und Diastereoisomeren, einschließlich deren razemischen Gemischen.

7. Verbindungen nach Anspruch 1, worin B für eine -NR4R5-Gruppe steht, wobei R4 und R5 unabhängig voneinander für eine Alkyl-, Cycloalkyl-, Hydroxyalkyl-oder Fluoralkylgruppe stehen, oder aber R4 und R5 mit dem Stickstoffatom, an das sie gebunden sind, einen 5-oder 6-gliedrigen Ring, wie etwa einen Pyrrolidinyl-oder Piperidinylring bilden, der gegebenenfalls mit einer Alkylgruppe substituiert ist,
deren Salzen, deren Enantiomeren und Diastereoisomeren, einschließlich deren razemischen Gemischen.

8. Verbindungen nach Anspruch 1, worin: X und Y unabhängig voneinander für ein Stickstoffatom oder ein -CR3-Glied stehen, wobei R3 für ein Wasserstoffatom oder eine Alkoxygruppe steht;
U für ein Wasserstoffatom oder eine NHR7-Gruppe steht, wobei R7 ein Wasserstoffatom oder eine Alkylgruppe ist;
A für eine Aryl-, Heteroaryl- oder Heterocycloalkylgruppe steht, ausgewählt aus den Phenyl-, Benzodioxolyl-, Thienyl-, Thiazolyl-, Pyridinyl-, Pyrazolyl- und Pyrrolidinongruppen, wobei die Aryl- oder Heteroarylgruppe gegebenenfalls an beliebigen Positionen mit einer oder mehreren Gruppen, ausgewählt aus einem Halogenatom und den Cyano-, Alkyl-, Halogenalkyl-, Hydroxy-, Alkoxy-, -O-(CH₂)ₚ-O-Alkyl-, Halogenalkoxy-, -NRR'-, -NR-CO-Alkyl- und -SO₂-Alkylgruppen substituiert ist, wobei R und R' unabhängig voneinander für ein Wasserstoffatom oder eine Alkylgruppe stehen und p eine ganze Zahl im Bereich zwischen 1 und 5 ist;
W für ein Halogenatom, eine Alkylgruppe oder eine Halogenalkylgruppe steht;
Z für eine Bindung oder eine Alkylengruppe steht, die gegebenenfalls mit mindestens einer Grupp, ausgewählt aus einem Halogenatom und den Alkyl- und Hydroxygruppen, substituiert ist;
B für Folgendes steht:
- entweder eine -NR4R5-Gruppe, wobei R4 und R5 unabhängig voneinander für eine Alkyl-, Hydroxyalkylgruppe stehen, oder aber R4 und R5, mit dem Stickstoffatom, an das sie gebunden sind, einen 5- oder 6-gliedrigen Ring, wie etwa einen Pyrrolidinyl- oder Piperidinylring, bilden,
- oder einen Heterocyclus mit der folgenden Formel: 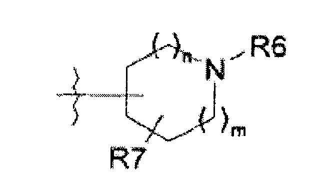
wobei m und n unabhängig voneinander für 0, 1 oder 2 stehen, und wobei R6 und R7 unabhängig voneinander für ein Wasserstoffatom oder eine Alkyl- oder Cycloalkylgruppe stehen;
deren Salzen, deren Enantiomeren und Diastereoisomeren, einschließlich deren razemischen Gemischen.

9. Verbindungen nach Anspruch 1, worin:
X und Y unabhängig voneinander für ein Stickstoffatom oder eine CH-Gruppe stehen;
A für eine Phenyl-, Pyridinyl- oder Pyrrolidinongruppe steht, die an beliebigen Positionen mit 1 oder 2 Gruppen, ausgewählt aus einem Halogenatom und den Alkyl-, Trifluormethyl-, Methoxy- und N,N-Dimethylamingruppen, substituiert ist;
U für ein Wasserstoffatom oder eine NHR7-Gruppe steht, wobei R7 ein Wasserstoffatom ist;
W für ein Chloratom oder eine Trifluormethylgruppe steht;
Z für eine Bindung oder eine Alkylengruppe steht, die gegebenenfalls mit einer Methylgruppe substituiert ist;
B für Folgendes steht:
- entweder eine -NR4R5-Gruppe, wobei R4 und R5 unabhängig voneinander für eine Alkylgruppe stehen, oder aber R4 und R5 mit dem Stickstoffatom, an das sie gebunden sind, einen 5- oder 6-gliedrigen Ring bilden,
- oder Heterocyclen mit der folgenden Formel: 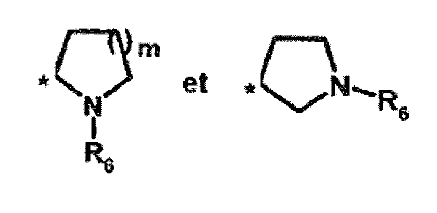
wobei m = 1 oder 2 und R6 für eine Ethyl- oder Methylgruppe steht;
deren Salzen, deren Enantiomeren und Diastereoisomeren, einschließlich deren razemischen Gemischen.

10. Verbindungen nach mindestens einem der vorhergehenden Ansprüche, worin:
X für ein Stickstoffatom und Y für eine CH-Gruppe steht;
A für eine Phenyl- oder Pyridinylgruppe steht, die an den Positionen 2, 4, 5 und 6 mit einer oder zwei Gruppen, ausgewählt aus einem Halogenatom, wie etwa Chlor oder Fluor, und den Alkylgruppen, wie etwa Methyl, Ethyl oder Isopropyl, Trifluormethyl, Methoxy und N,N-Dimethylamin, substituiert ist;
U für ein Wasserstoffatom oder eine NHR7-Gruppe steht, wobei R7 ein Wasserstoffatom ist;
W für ein Chloratom oder eine Trifluormethylgruppe steht;
Z für eine Bindung oder eine Ethylen-, Propylen-oder Methylpropylengruppe steht;
B für Folgendes steht:
- entweder eine -NR4R5-Gruppe, wobei R4 und R5 unabhängig voneinander für eine Methyl-, Ethyl- oder Propylgruppe stehen, oder zusammen mit dem Stickstoffatom, an das sie gebunden sind, einen 5- oder 6-gliedrigen Ring bilden,
- oder einen Heterocyclus mit der folgenden Formel: 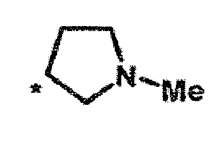
deren Salzen, deren Hydraten oder Solvaten sowie deren Enantiomeren und Diastereoisomeren, einschließlich deren razemischen Gemischen.

11. Verbindungen nach mindestens einem der vorhergehenden Ansprüche, worin:
X für ein Stickstoffatom und Y für eine CH-Gruppe steht;
A für eine Phenyl- oder Pyridinylgruppe steht, die an den Positionen 2, 4, 5 und 6 mit einer oder zwei Gruppen, ausgewählt aus einem Halogenatom, wie etwa Chlor oder Fluor, und den Alkylgruppen, wie etwa Methyl, Ethyl oder Isopropyl, Trifluormethyl, Methoxy und N,N-Dimethylamin, substituiert ist;
U für ein Wasserstoffatom oder eine NHR7-Gruppe steht, wobei R7 ein Wasserstoffatom ist;
W für ein Chloratom oder eine Trifluormethylgruppe steht;
Z für eine Bindung oder eine Ethylen-, Propylen-oder Methylpropylengruppe steht;
B für Folgendes steht:
- eine -NR4R5-Gruppe, wobei R4 und R5 unabhängig voneinander für eine Methyl-, Ethyl- oder Propylgruppe stehen, oder zusammen mit dem Stickstoffatom, an das sie gebunden sind, einen 5- oder 6-gliedrigen Ring bilden,
deren Salzen, deren Hydraten oder Solvaten sowie deren Enantiomeren und Diastereoisomeren, einschließlich deren razemischen Gemischen.

12. Verbindung nach mindestens einem der vorhergehenden Ansprüche, **dadurch gekennzeichnet, dass** sie ein Hydrochlorid ist.

## Claims

1. Compounds corresponding to formula (IV): 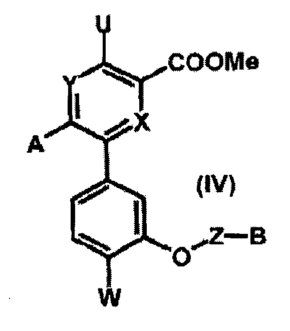 in which:
X and Y represent, independently of one another, a nitrogen atom or a -CR3- group, where R3 represents a hydrogen or halogen atom or an alkyl or alkoxy group;
U represents a hydrogen atom or a group NHR7, where R7 is a hydrogen atom or an alkyl group;
A represents an aryl, heteroaryl or heterocycloalkyl group optionally substituted in any positions with one or more groups selected from a halogen atom and the cyano, alkyl, haloalkyl, hydroxy, alkoxy, -O-(CH₂)ₚ-O-alkyl, haloalkoxy, -NRR', -NR-CO-alkyl, -SO- and -SO₂-alkyl groups, where R and R' represent, independently of one another, a hydrogen atom or an alkyl group and p is an integer between 1 and 5 and more particularly between 1 and 3;
W represents a halogen atom, an alkyl group or a haloalkyl group;
Z represents a bond, a cycloalkylene group or an alkylene group optionally substituted with one or more groups selected from a halogen atom and the alkyl, hydroxy and alkoxy groups;
B represents:
• either a group -NR4R5, where R4 and R5 represent, independently of one another, an alkyl, cycloalkyl, hydroxyalkyl or fluoroalkyl group, or alternatively R4 and R5 form, with the nitrogen atom to which they are attached, a 5- or 6-membered ring, such as a pyrrolidinyl or piperidinyl ring, optionally substituted with an alkyl group,
• or a heterocycle of the following formula: 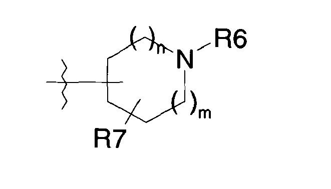
where m and n represent, independently of one another, 0, 1 or 2, and where R6 and R7 represent, independently of one another, a hydrogen atom or an alkyl or cycloalkyl group;
their salts, their enantiomers and diastereoisomers, including their racemic mixtures.

2. Compounds according to Claim 1, in which X and Y represent, independently of one another, a nitrogen atom or a -CR3- group, where R3 represents a hydrogen or halogen atom or an alkyl or alkoxy group;
their salts, their enantiomers and diastereoisomers, including their racemic mixtures.

3. Compounds according to Claim 1, in which U represents a hydrogen atom or a group NHR7, where R7 is a hydrogen atom or an alkyl group;
their salts, their hydrates or solvates as well as their enantiomers and diastereoisomers, including their racemic mixtures.

4. Compounds according to Claim 1, in which A represents an aryl, heteroaryl or heterocycloalkyl group selected from the phenyl, benzodioxolyl, thienyl, thiazolyl, pyridinyl, pyrimidinyl, pyridazinyl, pyrazinyl, pyrazolyl and pyrrolidinone groups, said aryl, heteroaryl or heterocycloalkyl group being optionally substituted;
their salts, their hydrates or solvates as well as their enantiomers and diastereoisomers, including their racemic mixtures.

5. Compounds according to Claim 1, in which W represents a halogen atom, an alkyl group or a haloalkyl group;
their salts, their enantiomers and diastereoisomers, including their racemic mixtures.

6. Compounds according to Claim 1, in which Z represents a bond, a cycloalkylene group or an alkylene group optionally substituted with one or more groups selected from a halogen atom and the alkyl, hydroxy and alkoxy groups;
their salts, their enantiomers and diastereoisomers, including their racemic mixtures.

7. Compounds according to Claim 1, in which B represents a group -NR4R5, where R4 and R5 represent, independently of one another, an alkyl, cycloalkyl, hydroxyalkyl or fluoroalkyl group, or alternatively R4 and R5 form, with the nitrogen atom to which they are attached, a 5- or 6-membered ring, such as a pyrrolidinyl or piperidinyl ring, optionally substituted with an alkyl group,
their salts, their enantiomers and diastereoisomers, including their racemic mixtures.

8. Compounds according to Claim 1, in which:
X and Y represent, independently of one another, a nitrogen atom or a -CR3- group, where R3 represents a hydrogen atom or an alkoxy group;
U represents a hydrogen atom or a group NHR7, where R7 is a hydrogen atom or an alkyl group;
A represents an aryl, heteroaryl or heterocycloalkyl group selected from the phenyl, benzodioxolyl, thienyl, thiazolyl, pyridinyl, pyrazolyl and pyrrolidinone groups, said aryl or heteroaryl group being optionally substituted in any positions with one or more groups selected from a halogen atom and the cyano, alkyl, haloalkyl, hydroxy, alkoxy, -O-(CH₂)ₚ-O-alkyl, haloalkoxy, -NRR', -NR-CO-alkyl and -SO₂-alkyl groups, where R and R' represent, independently of one another, a hydrogen atom or an alkyl group and p is an integer between 1 and 5;
W represents a halogen atom, an alkyl group or a haloalkyl group;
Z represents a bond or an alkylene group optionally substituted with at least one group selected from a halogen atom and the alkyl and hydroxy groups;
B represents:
• either a group -NR4R5, where R4 and R5 represent, independently of one another, an alkyl, hydroxyalkyl group, or alternatively R4 and R5 form, with the nitrogen atom to which they are attached, a 5- or 6-membered ring, such as a pyrrolidinyl or piperidinyl ring,
• or a heterocycle of the following formula: 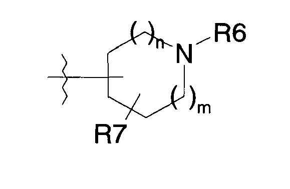
where m and n represent, independently of one another, 0, 1 or 2, and where R6 and R7 represent, independently of one another, a hydrogen atom or an alkyl or cycloalkyl group;
their salts, their enantiomers and diastereoisomers, including their racemic mixtures.

9. Compounds according to Claim 1, in which:
X and Y represent, independently of one another, a nitrogen atom or a CH group;
A represents a phenyl, pyridinyl, or pyrrolidinone group, substituted in any positions with 1 to 2 groups selected from a halogen atom, and the alkyl, trifluoromethyl, methoxy and N,N-dimethylamine groups;
U represents a hydrogen atom or a group NHR7, where R7 is a hydrogen atom;
W represents a chlorine atom or a trifluoromethyl group;
Z represents a bond or an alkylene group optionally substituted with a methyl group;
B represents:
• either a group -NR4R5, where R4 and R5 represent, independently of one another, an alkyl group or alternatively R4 and R5 form, with the nitrogen atom to which they are attached, a 5- or 6-membered ring,
• or heterocycles of the following formula: 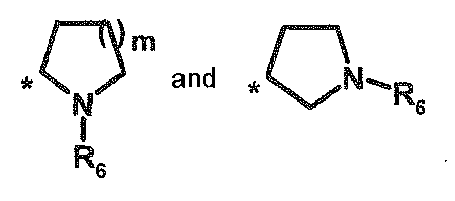
where m = 1 or 2 and R6 represents an ethyl or methyl group;
their salts, their enantiomers and diastereoisomers, including their racemic mixtures.

10. Compounds according to at least one of the preceding claims, in which:
X represents a nitrogen atom and Y represents a CH group;
A represents a phenyl or pyridinyl group, substituted in positions 2, 4, 5 and 6 by one or two groups selected from a halogen atom, such as chlorine or fluorine, and the alkyl groups, such as methyl, ethyl or isopropyl, trifluoromethyl, methoxy and N,N-dimethylamine;
U represents a hydrogen atom or a group NHR7, where R7 is a hydrogen atom;
W represents a chlorine atom or a trifluoromethyl group;
Z represents a bond or an ethylene, propylene or methylpropylene group;
B represents:
• either a group -NR4R5, where R4 and R5 represent, independently of one another, a methyl, ethyl or propyl group or form together with the nitrogen atom to which they are attached a 5- or 6-membered ring,
• or a heterocycle of the following formula: 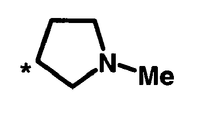
their salts, their hydrates or solvates as well as their enantiomers and diastereoisomers, including their racemic mixtures.

11. Compounds according to at least one of the preceding claims, in which:
X represents a nitrogen atom and Y represents a CH group;
A represents a phenyl or pyridinyl group, substituted in positions 2, 4, 5 and 6 by one or two groups selected from a halogen atom, such as chlorine or fluorine, and the alkyl groups, such as methyl, ethyl or isopropyl, trifluoromethyl, methoxy and N,N-dimethylamine;
U represents a hydrogen atom or a group NHR7, where R7 is a hydrogen atom;
W represents a chlorine atom or a trifluoromethyl group;
Z represents a bond or an ethylene, propylene or methylpropylene group;
B represents:
• a group -NR4R5, where R4 and R5 represent, independently of one another, a methyl, ethyl or propyl group or form together with the nitrogen atom to which they are attached a 5- or 6-membered ring,
their salts, their hydrates or solvates as well as their enantiomers and diastereoisomers, including their racemic mixtures.

12. Compound according to at least one of the preceding claims, **characterized in that** it is a hydrochloride.
